# EUROPEAN PATENT APPLICATION

(11) **EP 4 241 793 A1**
(43) Date of publication of application: **13.09.2023**
(21) Application number: 22160908.4
(22) Date of filing: 08.03.2022
(51) Int. Cl.: A61K 49/12, A61K 49/18

(54) **GLOBULAR NANOSTRUCTURES**

(71) Applicant: Spago Nanomedical AB, 223 63 Lund (SE)
(72) Inventor: AXELSSON, Oskar, 243 32 HÖÖR (SE); EKENGARD, Erik, 212 31 MALMÖ (SE); LIU, Yi-Chi, 224 71 LUND (SE); LARSSON, Rikard, 268 75 TÅGARP (SE); BÄCKSTRÖM, Sania, 275 62 BLENTARP (SE)
(74) Representative: Ström & Gulliksson AB

(57) **Abstract**

The present disclosure relates to a plurality of nanostructures having an average hydrodynamic diameter between 20 to 50 nm, wherein each nanostructure is a polymeric nanostructure comprising a central part, an anchoring layer surrounding the central part and a coating layer surrounding the anchoring layer. The present disclosure also relates to such a plurality of nanostructures for use as a medicament, especially for use in the treatment of cancer and/or imaging, as well as the use such nanostructures as carriers of radionuclides. The present disclosure also relates to a method for radiolabeling such nanostructures with a multivalent cationic radionuclide and to a kit.

## Description

### TECHNICAL FIELD

The present disclosure relates to bioinert, chelating polymeric nanostructures with applications in systemic radiotherapy and cancer imaging.

### BACKGROUND

The gold standard for cancer treatment is surgery. In cases where surgery alone is not curative, multimodality regimens including chemotherapy and radiation treatment are used. Although the intensity, location and timing for external radiation can be well controlled and modulated, disadvantages associated with this technique include the destruction of normal tissue in the path of the beam as well as damage to tissues surrounding the tumor. The risk of damaging surrounding healthy tissue speaks against external radiotherapy to deeply situated tumors and tumors situated next to vital organs. Furthermore, high radiation doses are frequently required to penetrate the tissue. Moreover, in order to be efficient, external radiotherapy often requires the patients to submit themselves to daily hospital visits over extended periods of time.

Systemic radiotherapy, which internally delivers radioactive substances to the tumor, offers solutions to many of the above-mentioned disadvantages connected with external radiotherapy.

The most commonly used radionuclides for systemic radiotherapy in clinics today are beta-emitters. Beta-emitters with energies between 0.1-2.2 MeV are ideal for the treatment of small to large clusters of tumor cells. The maximum tissue penetration range (1-10 mm) and cross-fire effects, i.e. the ability to kill cells indirectly along a longer path length, of beta-particles of such energies thus allows for the targeting of tumor cells in close proximity to neovasculature. Radionuclides such as ¹³¹I are used alone, as in the treatment of thyroid cancers, or conjugated with monoclonal antibodies or peptides to allow for tumor-targeting radioimmunotherapy. Ibritumomab tiuexan with ⁹⁰Y (Zevalin^{®}) and tositumomab coupled with ¹³¹I (Bexxar^{®}) are two examples of approved radioimmunotherapy regimens both targeting B lymphocytes to treat B-cell non-Hodgkin lymphoma.

Clinical use of alpha-emitters is less common, but some show clinical potential, such as the alpha-emitter ²²³Ra (Xofigo^{®}).

Most use of radionuclides for systemic radiotherapy requires the use of a carrier as the radionuclides as atoms or simple salts do not have favorable pharmaceutical properties.

Recent advances in nanotechnology have led to the development of novel nanocarriers designed for cancer detection and screening, *in vivo* molecular and cellular imaging as well as the delivery of therapeutics. However, despite a large number of publications covering nanosized carriers for cancer therapeutics, relatively few have reached clinical trials, and only a handful are approved by the FDA.

Several approaches, involve biodegradable materials which allow for fast or slow release of the carried therapeutic agent.

WO 2004/040972 discloses microspheres comprising a polymeric hydrogel associated with radioactive particles. The microspheres are larger than 100 nm.

EP2923712A1 discloses a globular nanostructure having a hydrodynamic diameter (D h) of 8-100 nm comprising a central part and a peripheral part. The nanostructures may comprise a radionuclide chelated to the nanostructures and may be used in imaging and/or radiotherapy.

Many of the known approaches to radioisotope therapy involving alleged nanocarriers suffer from the drawback that the nanocarrier is not really a nanocarrier as it is larger than 100 nm and due to its large size suffers from the drawback of not delivering the radioisotope to the tumor tissue in an effective way. Further, many of the known nanocarriers are only stable for a short period of time and/or do not bind the radionuclide in a sufficiently strong manner. It is an object of the present disclosure to solve these problems.

### SUMMARY

According to a first aspect, the above and other objectives are achieved, in full or in part by a plurality of globular nanostructures as defined by claim 1. According to this claim, the above object is achieved by a plurality of globular nanostructures having an average hydrodynamic diameter between 20 to 50 nm, wherein each nanostructure is a polymeric nanostructure comprising:
a central part comprising monomer residues according to Formula (I): 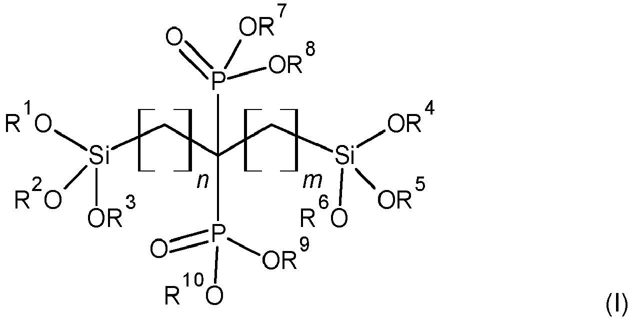 wherein
   - each R¹, R², R³, R⁴, R⁵, and R⁶ is independently chosen from the group consisting of H, a negative charge, and a covalent bond;
   - each R⁷, R⁸, R⁹, and R¹⁰ is independently chosen from the group consisting of H, a negative charge, and lower alkyls, wherein at least 95 % of all of the R⁷, R⁸, R⁹, and R¹⁰ groups are H or a negative charge;
   - n is an integer between 2 and 5; and
   - m is an integer between 2 and 5;
an anchoring layer surrounding the central part, wherein the anchoring layer comprises monomer residues according to Formula (II): 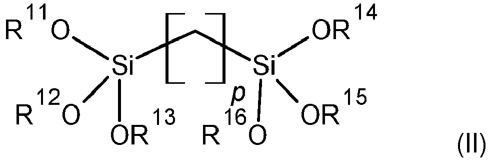 wherein
   - each R¹¹, R¹², R¹³, R¹⁴, R¹⁵, and R¹⁶ is independently chosen from the group consisting of H, a negative charge, and a covalent bond; and
   - p is an integer between 1 and 3;
   and
a coating layer surrounding the anchoring layer, wherein the coating layer comprises monomer residues according to Formula (III): 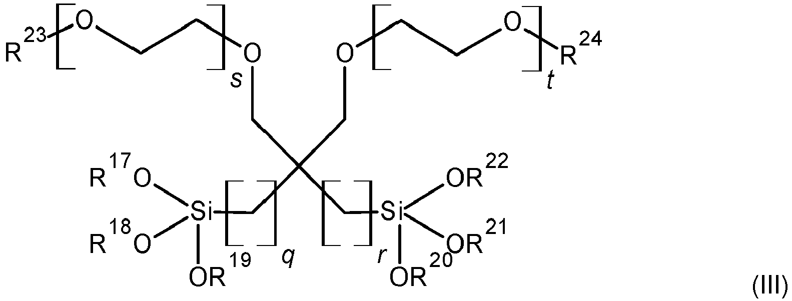 wherein
   - each R¹⁷, R¹⁸, R¹⁹, R²⁰, R²¹, and R²² is independently chosen from the group consisting of H, a negative charge, and a covalent bond;
   - q is an integer between 2 and 5;
   - r is an integer between 2 and 5;
   - each R²³ and R²⁴ is independently chosen from the group consisting of H and lower alkyls;
   - s is an integer between 30 and 105; and
   - t is an integer between 30 and 105.

The covalent bonds are covalent bonds to the rest of the nanostructure.

Such nanostructures are strong chelators of multivalent metal ions, and are highly biocompatible.

The core (also referred to as core nanostructure or central part) comprises chelating bisphosphonate groups that are able to chelate multivalent metal ions.

The anchoring layer joins the core to the coating layer.

The coating layer protects the nanostructures from aggregation and renders the nanostructures highly biocompatible.

The nanostructures of the present disclosure have hydrodynamic diameters between 20 and 50 nm. Nanostructures of such a size are suitable for targeting of tumors through the EPR effect.

Preferably, the nanostructures have hydrodynamic diameters between 21 and 40 nm. It is advantageous to use nanostructures having hydrodynamic diameters between 22 and 37 nm, as this results in strong accumulation of the nanostructures in tumor tissue. Thus, the hydrodynamic diameter of the nanostructures may preferably between 23 and 35 nm, such as between 25 and 30 nm.

Typically, the dispersity of the plurality of nanostructures is low, such as below 1.6, such as between 1 and 1.6, or between 1 and 1.4, or between 1.1 and 1.3, or between 1.13 and 1.27 as measured by DLS.

Preferably, the dispersity is below 1.4, such as below 1.3, such as below 1.2.

Preferably, n and m are independently 2 or 3. Even more preferably, n and m are both 3.

Preferably, p is 1 or 2. Even more preferably, p is 1.

Preferably, q and r are both 3.

Preferably, s and t are both between 35 and 55, or both between 45 and 65, or both between 60 and 80.

Preferably, R²³ and R²⁴ and are both methyl or ethyl.

Preferably, q and r are both 3, s and t are both between 35 and 55, and R²³ and R²⁴ and are both methyl.

According to one embodiment, X_{CP}, denoting the percentage of the number of monomer residues of the central part in relation to the total number of monomer residues of the nanostructure, is between 5% and 58%; X_{AL}, denoting the percentage of the number of monomer residues of the anchoring layer in relation to the total number of monomer residues of the nanostructure, is between 39% and 93%; X_{CL}, denoting the percentage of the number of monomer residues of the coating layer in relation to the total number of monomer residues of the nanostructure, is between 0.75% and 4.5%; and wherein 70% < (X_{CP} + X_{AL} + X_{CL}) ≤ 100%.

Preferably, monomer residues according to Formula (I) make up at least 70% of the monomer residues of the central part and/or monomer residues according to Formula (II) make up at least 70% of the monomer residues of the anchoring layer and/or monomer residues according to Formula (III) make up at least 70% of the monomer residues of the coating layer.

According to another embodiment, the average hydrodynamic diameter is from 22 to 37 nm; n = 3; m = 3; p = 1; q = 3; r = 3; R²³ and R²⁴ are independently chosen from the group consisting of lower alkyls; and/or X_{CP}, denoting the percentage of the number of monomer residues of the central part in relation to the total number of monomer residues of the nanostructure, is between 20% and 40%; X_{AL}, denoting the percentage of the number of monomer residues of the anchoring layer in relation to the total number of monomer residues of the nanostructure, is between 60% and 85%; X_{CL}, denoting the percentage of the number of monomer residues of the coating layer in relation to the total number of monomer residues of the nanostructure, is between 1.5% and 4%; and wherein 81.5% < (X_{CP} + X_{AL} + X_{CL}) ≤ 100%.

Preferably the lower alkyls are methyl and/or ethyl.

Thus, the average hydrodynamic diameter may be from 22 to 37 nm; n = 3; m = 3; p = 1; q = 3; r = 3; R²³ and R²⁴ are independently chosen from the group consisting of lower alkyls.

Preferably, X_{CP}, denoting the percentage of the number of monomer residues of the central part in relation to the total number of monomer residues of the nanostructure, is between 20% and 40%; X_{AL}, denoting the percentage of the number of monomer residues of the anchoring layer in relation to the total number of monomer residues of the nanostructure, is between 60% and 85%; X_{CL}, denoting the percentage of the number of monomer residues of the coating layer in relation to the total number of monomer residues of the nanostructure, is between 1.5% and 4%; and wherein 81.5% < (X_{CP} + X_{AL} + X_{CL}) ≤ 100%.

Preferably, the average hydrodynamic diameter is from 22 to 37 nm; n = 3; m = 3; p = 1; q = 3; r = 3; R²³ and R²⁴ are independently chosen from the group consisting of lower alkyls; and X_{CP}, denoting the percentage of the number of monomer residues of the central part in relation to the total number of monomer residues of the nanostructure, is between 20% and 40%; X_{AL}, denoting the percentage of the number of monomer residues of the anchoring layer in relation to the total number of monomer residues of the nanostructure, is between 60% and 85%; X_{CL}, denoting the percentage of the number of monomer residues of the coating layer in relation to the total number of monomer residues of the nanostructure, is between 1.5% and 4%; and wherein 81.5% < (X_{CP} + X_{AL} + X_{CL}) ≤ 100%.

According to a further embodiment, at least 50 % of all of the R¹, R², R³, R4, R⁵, R6, R¹¹, R¹², R¹³, R¹⁴, R¹⁵, R¹⁶ R¹⁷, R¹⁸, R¹⁹, R²⁰, R²¹, and R²² groups are covalent bonds. The covalent bonds are covalent bonds to the rest of the polymeric structure.

According to yet another embodiment, the globular nanostructures further comprise one or more radionuclides. Such nanostructures can be used in treatment of cancer and/or imaging.

The radionuclide may be a radionuclide suitable for imaging, such as PET imaging or SPECT imaging.

The radionuclide for PET imaging may be gallium-68 (⁶⁸Ga).

The radionuclide for SPECT imaging may be technetium-99m in its tri-cationic form (^{99m}Tc³⁺).

The radionuclide may be a radionuclide suitable for radiotherapy and/or imaging.

(¹⁷⁷Lu). radionuclide for imaging and/or radiotherapy may be lutetium-177

The radionuclide for imaging and/or radiotherapy may be yttrium-90 (⁹⁰Y).

A plurality of nanostructures may carry on average from 0.5 to 2 radioisotopes per nanostructure.

According to a further embodiment, the radionuclide is ¹⁷⁷Lu.

According to a second aspect, there is provided a plurality of globular nanostructures according to the present disclosure for use as a medicament.

Notably, both nanostructures comprising a radionuclide and nanostructures comprising a non-radioactive isotope as well as empty nanostructures, i.e. nanostructures not chelating multivalent metal ions, can be used as a medicament.

According to a third aspect, there is provided a plurality of globular nanostructures according to the present disclosure and comprising a radionuclide for use in the treatment of cancer and/or imaging.

According to a fourth aspect, there is provided the use of a plurality of globular nanostructures according to the present disclosure as carriers of radionuclides. In these cases, the nanocarriers are "empty" as described above.

According to a fifth aspect, there is provided a pharmaceutical composition comprising a plurality of globular nanostructures according to the present disclosure, water, and at least one excipient.

The at least one excipient may be a stabilizer, an antioxidant, a cryoprotectant, a preservative, and/or an antibiotic.

The pharmaceutical composition may comprise more than one excipient.

The pharmaceutical composition may comprise an antioxidant.

The antioxidant is preferably thioglycerol and/or gentisic acid. Nanostructures formulated with thioglycerol and/or gentisic acid do not risk generating calcium oxalate precipitates when administered into a patient together with calcium.

The pharmaceutical composition may comprise thioglycerol and gentisic acid.

In some cases, the pharmaceutical composition comprises an antioxidant other than a sulphite or an ascorbate. Compositions comprising a sulphite or an ascorbate.

In some cases, the antioxidant is not sodium metabisulphite. Sodium metabisulphite suppresses the degradation of the nanostructures through oxidation of polyethylene glycol chains in the coating layer. Surprisingly, it has been found that sodium metabisulphite interferes with the binding of metal ions to the nanostructures, making sodium metabisulphite a less suitable excipient for formulations of nanostructures according to the present disclosure.

The pharmaceutical composition may comprise a cryoprotectant. The cryoprotectant is preferably glycerol.

The pharmaceutical composition may comprise an antioxidant and a cryoprotective.

The pharmaceutical composition may comprise two antioxidants and one cryoprotective.

The pharmaceutical composition may comprise thioglycerol, gentisic acid and glycerol.

According to one embodiment, the pharmaceutical composition further comprises 0.1 to 10 mg/ml thioglycerol; 0.03 to 3 mg/ml gentisic acid; and 3 to 300 mg/ml glycerol.

According to a sixth aspect, there is provided a pharmaceutical composition according to the present disclosure for use as a medicament.

According to a seventh aspect, there is provided a pharmaceutical composition according to the present disclosure for use in the treatment of cancer and/or imaging, wherein the pharmaceutical composition comprises a plurality of globular nanostructures according to the present disclosure comprising a radionuclide.

According to an eighth aspect, there is provided a method for radiolabeling a plurality of globular nanostructures with a multivalent cationic radionuclide, wherein the method comprises the steps of:
a) providing a solution having a pH of below 3.5 and comprising a plurality of globular nanostructures, preferably wherein the nanostructures are nanostructures according to any one of claims 1 to 4;
b) contacting the solution of step a) with a pharmaceutically acceptable salt of the radionuclide; and
c) adjusting the pH of the solution to above 6.

Notably, the same method steps can be used to load nanostructures with non-radioactive multivalent metal ions.

Preferably, the solution in step a) is an aqueous solution.

The solution in step a) may further comprise one or more excipients, such as a stabilizer, an antioxidant, a cryoprotectant, a preservative, and/or an antibiotic.

In step a), the solution preferably has a pH below 3.

The pharmaceutically acceptable salt of the radionuclide used in step b) is typically used as a solution of the radioisotope. Contacting the solution of nanostructures with the radioisotope can be realized through the mixing of the solution of the nanostructures and the solution of the radioisotope.

The radionuclide in step b) may be used in an amount from 0.01 to 2 atom(s) of the radionuclide per nanostructure, such as from 0.5 to 1 atom of the radionuclide per nanostructure.

It is advantageous to incubate the solution for some time after step b) prior to performing step c). Preferably, this incubation is performed at slightly elevated temperature, such as between 40 °C and 80 °C, such as between 50 °C and 70 °C, such as between 50 °C and 60 °C. The incubation period is preferably at least 15 minutes, such as between 30 minutes and 3 hours.

Thus, step b) may further comprise, after contacting the solution of step a) with a pharmaceutically acceptable salt of the radionuclide, incubating the solution at a temperature between 40 °C and 80 °C for at least 15 minutes.

The adjustment of pH in step c) may be realized through addition of a buffer solution to the solution of the nanostructures. It is also conceivable to adjust the pH through the addition of a strong base, such as an alkali hydroxide or an alkaline earth metal hydroxide.

In step c), the pH may be adjusted by mixing the solution of step b) with an aqueous buffer having a pH above 6.

It is advantageous to incubate the solution for some time after step c) prior to use of the radiolabeled nanostructures. Preferably, this incubation is performed at slightly elevated temperature, such as between 40 °C and 80 °C, such as between 50 °C and 70 °C, such as between 50 °C and 60 °C. The incubation period is preferably at least 15 minutes, such as between 30 minutes and 3 hours.

The adjustment of pH in step c) may be performed by the addition of a buffer comprising Tris, with a pH above 7.5.

The solution may be incubated at between 50 °C and 70 °C for between 30 minutes and 2 hours between step b) and step c).

The solution may be incubated at between 50 °C and 70 °C for between 1 hour and 3 hours after step c) before the radiolabeled nanostructures are used.

In one embodiment, in step a), the solution has a pH below 3; and/or step b) further comprises, after contacting the solution of step a) with a pharmaceutically acceptable salt of the radionuclide, incubating the solution at a temperature between 40 °C and 80 °C for at least 15 minutes; and/or in step c), the pH is adjusted by mixing the solution of step b) with an aqueous buffer having a pH above 6.

According to a ninth aspect, there is provided a kit, comprising an aqueous solution of globular nanostructures according to any one of claims 1 to 4, having a pH below 3.5; an aqueous solution of a pharmaceutically acceptable buffer having a pH of at least 6; and instructions for use, allowing for the realization of the method of radiolabeling according to the present disclosure.

Optionally, the kit further comprises a mixing vessel; an apparatus for sterile filtration; a pharmaceutically acceptable dilutant; a centrifugal filter; and equipment for the shielding of ionizing radiation.

The kit can be used in a radiopharmacy to produce one or a small number of doses of radiolabeled nanostructures for use in the imaging and/or treatment of a pathological condition such as cancer.

The kit can be used in combination with a source of radioisotope to generate radiolabeled nanostructures suitable for intravenous use.

The kit may further comprise a mixing vessel, suitable for the use in steps b) and c) of the method described above. The vessel may be of a size suitable to contain the solution of radiolabeled nanostructures as obtained at the end of the radiolabeling process, or it may be significantly larger, allowing for the dilution of the solution of radiolabeled nanostructures to a significantly larger volume. Such dilution may be advantageous if the radiolabeled nanostructures are to be administrated by slow infusion.

The kit may further comprise an apparatus for sterile filtration, such as syringe filters, syringes and receiving vessels.

The kit may further comprise a pharmaceutically acceptable dilutant, such as physiological saline solution (0.90% w/v of NaCl in water) or a calcium containing buffer.

The kit may further comprise equipment for quality control. Such equipment for quality control may compromise centrifugal filters, allowing for the quantification of the binding of the radioisotope through determination of the activity of the permeate. Such equipment for quality control may also comprise components necessary for the implementation of chromatographic methods, such as TLC plates, eluents, and sample preparation components for analysis by HPLC, suitable for the control of the identity and radiochemical purity of the radiolabeled nanostructures.

The kit may further comprise equipment for the shielding of ionizing radiation. Such equipment may comprise shielded containers, suitable to store and transport vessels for radioactive material in. Such shielded containers may be of a size and shape suitable to enclose containers that are part of the kit. Such equipment for the shielding of ionizing radiation may also include shielded equipment for the handling of liquids, such as shielded syringes.

The kit may also comprise secondary and tertiary enclosures, allowing the collection of the components of the kit into one or a small number of units, suitable for commercial storage and shipment of the kit. Such enclosures may comprise paper and/or plastic boxes, and/or plastic or paper bags. Such enclosures may be suitable for storage of the kit or parts of the kit in a freezer.

The kit may also comprise one or more identifiers, such as bar codes or RFID chips. Such identifiers may be present in or on the kit or in one or more of the vessels or containers included in the kit. An identifier can be used to uniquely identify the kit for purposes of quality control or inventory control.

The present disclosure also relates to the use of nanostructures according to the present disclosure as intermediates in the production of a pharmaceutical composition.

Other objectives, features and advantages of the present disclosure will appear from the following detailed description, from the experimental data, as well as from the attached claims. It is noted that the disclosure relates to all possible combination of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least on instance of said component, means, step, etc., unless explicitly stated otherwise. The steps of any method disclosed herein do not have to be performed in the exact order disclosed, unless explicitly stated.

As used herein, the term "compromising" and variations of that term are not intended to exclude other additives, components, excipients, integers or steps.

### DEFINITION OF TERMS

The term "nanostructure" as used herein relates to an entity with a total size in the nanorange, i.e. up to 100 nm. As used herein the term excludes the structures often referred to as "core-shell nanoparticles" or just "nanoparticles" which usually have an inorganic core and an organic coating.

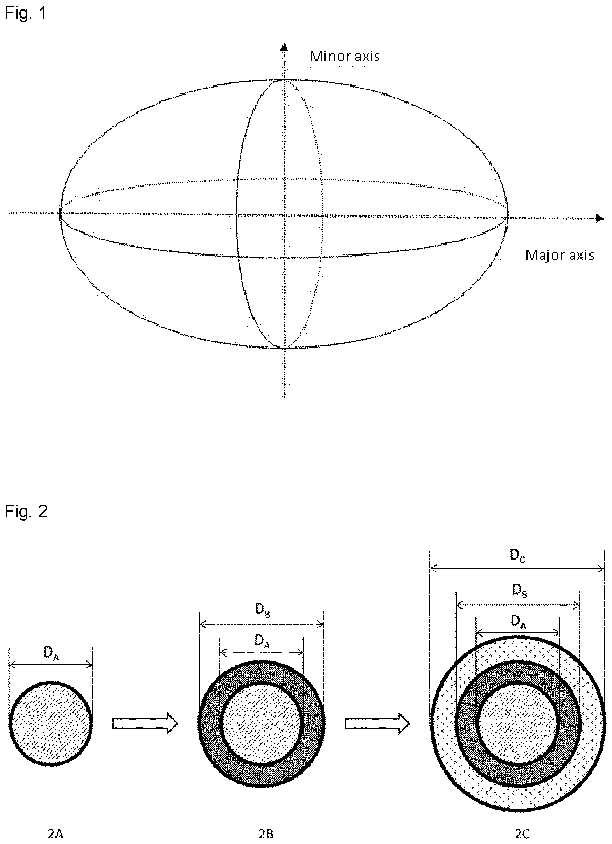

The term "globular" as used herein is meant to describe a shape such that the minor axis is no less than half of the major axis, i.e. the longest axis through the center (point of weight) of the structure is no more than twice the length of the shortest axis through the same point. For an explanatory illustration, not limiting this definition, see Fig. 1.

The term "globular nanostructure" as used herein relates to a nanostructure as discussed above having an essentially globular form or shape. This means that shapes such as flakes, rods, tubes, toroids, chains and ribbons are excluded.

The term "hydrodynamic diameter" as used herein refers to the diameter of the hypothetical hard sphere that diffuses at the same speed as the particle in solution, i. e. the diameter of the equivalent hard sphere as calculated from the diffusion coefficient, according to the Stokes-Einstein equation. The term is also known as "Stokes diameter" or "Stokes-Einstein diameter". Hydration and shape are included in the behavior of the sphere. The diffusion coefficient is in turn calculated from e.g. the time dependent light scattering data obtained by the Dynamic Light Scattering (DLS) technique. Other technical methods to measure the diffusion coefficient of nanostructures are known to one skilled in the art and may be used instead. In those cases, measurements need to be referenced to the DLS-measurement. As a comparison, bovine serum albumin is measured to have a hydrodynamic diameter of 6.5 nm by DLS in aqueous saline at pH 7 and room temperature. Depending on whether the number average, volume average, or scattered intensity average is used, the calculated values may be somewhat different. The volume average is generally the most useful since it shows which particle size the bulk of the material has. The average diameters(or average hydrodynamic diameters) referred to in this text refers to volume averages as measured in aqueous saline or 8% (v/v) aqueous ethylene glycol at pH 7 and room temperature.

The term "DLS" as used herein is an acronym for Dynamic Light Scattering, a particle sizing method, and may also be referred to as Photon Correlation Spectroscopy or Quasi-Elastic Light Scattering. The DLS sizes given as stated in the text and in the claims, if nothing else is specified, refers to the average of the volume weighted peak for a sample measured at 25 °C in aqueous solution with an ionic strength corresponding to 150 mM NaCl, also called saline, or to 8% (v/v) aqueous ethylene glycol.

The term "dispersity" as used herein is a measure of the width of the size distribution of a sample of macromolecules or nanostructures. For most applications it is preferable to have as narrow a distribution of sizes as possible around the average i.e. a low dispersity. For a sample with completely uniform size the dispersity is 1 and for non-uniform samples it will be larger than 1. The dispersity can be calculated in different ways, either as the ratio of M_{w}/Mₙ or similarly Mv/Mn, where Mw is the weight-average molecular weight, Mn is number-average molecular weight, and Mᵥ is the volume-average molecular weight. Herein we analogously define dispersity, Ð_{d}, for a sample of nanostructures as Ð_{d} = dᵥ/dₙ were dᵥ is the volume-average diameter as measured by DLS and dₙ is the number average diameter as measured by DLS.

A "monomer" is a molecule that may bind covalently to other molecules of the same kind, and optionally to other kinds, to form a polymer i.e. a macromolecule composed of several monomer residues. The term "monomer residue" refers to the atoms derived from one monomer unit as incorporated into the larger polymer. Depending on how the monomers link up, all the atoms in the monomer may be retained in the monomer residue or some may be lost as the bond forms.

A "crosslink" refers to a link between two different chains in a polymer. It is usually formed by the reaction of multifunctional monomers (i.e. crosslinkers) added when forming the polymer. Crosslinks may also be introduced e.g. by radiation treatment, chemical means, or heat. For the nanostructures of the present disclosure, which are network polymers of monomers with multiple branching capability the usual description of degree of crosslinking becomes somewhat unsuitable. For simplicity we just describe the average number of bonds between monomers. For example: if the monomer has the potential to form six bonds to other molecules and on average three of them are fulfilled, we avoid the term degree of crosslinking and state that we have "on average three bonds to other monomers" or sometimes "on average three bonds out of six possible to other monomers".

The term "cross-linked" refers to a structure formed after the formation of at least one crosslink.

The term "polymeric framework" as used herein relates to a covalently bound group of atoms forming a network structure with multiple crosslinks. Such polymeric frameworks are formed from the linking of suitable monomers and/or oligomers (i.e. a molecular complex consisting of a few monomer residues) via covalent bonds.

The term "degree of polymerization" is typically expressed as chain length or by the molecular weight of the polymer. The degree of polymerization can be measured by mass spectroscopy, gel filtration or dynamic lightscattering.

Herein, when the degree of polymerization is given as a range, It is understood by one skilled in the art that this signifies that a large portion, such as a majority of, such as more than 80 %, or such as more than 90 %, or such as more than 95 %, of the polyethylene glycol groups have degrees of polymerization falling within the given range. It is also understood that some small fraction of the polymer groups present can have degrees of polymerization that is outside of the given range, and that this might include both higher and lower degrees of polymerization than the given range. When the degree of polymerization is given as a single value it is understood, unless otherwise specified, that the value signifies the average or typical degree of polymerization.

The term "chelating group" refers to a chemical group with the ability to successfully compete with water in electrostatic binding of a positively charged ion. A single chelating group does not bind very strongly but if several chelating groups surround a positively charged ion, a synergistic strengthening of the binding occurs. This is called chelation.

The term "independently selected" as used herein in means that each of the different constituents mentioned before the term is selected from the group following after the term independently or separately from the selection of the other mentioned constituents.

The term "geminal bisphosphonate group" refers to two phosphonate groups separated by one carbon atom, i.e. the phosphonate groups are bound to the same carbon atom. Compounds comprising such a geminal bisphosphonate group are often referred to as 1,1 -bisphosphonates (or 1,1 - diphosphonates). The phosphonate groups in the geminal bisphosphonate group may be substituted.

The term "radionuclide" refers to an unstable form of a chemical element that decays radioactively, resulting in the emission of α, β and/or y radiation.

As used herein, the expression "radionuclides for imaging and/or radiotherapy" refers to actinium-225 (²²⁵Ac); copper-62 (⁶²Cu); copper-64 (⁶⁴Cu); copper-67 (⁶⁷Cu); gallium-67 (⁶⁷Ga); gallium-68 (⁶⁸Ga); holmium-166 (¹⁶⁶Ho); indium-111 (¹¹¹In); lead-212 (²¹²Pb); lutetium-177 (¹⁷⁷Lu); radium-223 (²²³Ra); rhenium-186 (¹⁸⁶Re); rhenium-188 (¹⁸⁸Re); rubidium-82 (⁸²Rb); samarium-153 (¹⁵³Sm); strontium-89 (⁸⁹Sr); technetium-99m (^{99m}Tc³⁺); thallium-201 (²⁰¹TI); thorium-227 (²²⁷Th); yttrium-86 (⁸⁶Y); yttrium-90 (⁹⁰Y); and zirconium-89 (⁸⁹Zr). The expression "a radionuclide for imaging and/or radiotherapy" also encompasses combinations of two or more of the above mentioned radionuclides.

As used herein, the expression "radionuclides for imaging" refers to copper-62 (⁶²Cu); copper-67 (⁶⁷Cu); gallium-67 (⁶⁷Ga); gallium-68 (⁶⁸Ga); indium-111 (¹¹¹In); lutetium-177 (¹⁷⁷Lu); rhenium-186 (¹⁸⁶Re); rubidium-82 (⁸²Rb): technetium-99m (^{99m}Tc³⁺); Thallium-201 (²⁰¹TI); yttrium-86 (⁸⁶Y) and zirconium-89 (⁸⁹Zr). The expression "a radionuclide for imaging" also encompasses combinations of two or more of the above mentioned radionuclides.

As used herein, the expression "radionuclides for PET imaging" refers to copper-62 (⁶²Cu); gallium-68 (⁶⁸Ga); rubidium-82 (⁸²Rb); yttrium-86 (⁸⁶Y) and zirconium-89 (⁸⁹Zr). The expression "a radionuclide for PET imaging" also encompasses combinations of two or more of the above mentioned radionuclides.

As used herein, the expression "radionuclides for SPECT imaging" refers to gallium-67 (⁶⁷Ga); indium-111 (¹¹¹In); technetium-99m (^{99m}Tc³⁺), lutetium-177 (¹⁷⁷Lu), and thallium-201 (²⁰¹TI). The expression "a radionuclide for SPECT imaging" also encompasses combinations of two or more of the above mentioned radionuclides.

As used herein, the expression "radionuclides for radiotherapy" refers to actinium-225 (²²⁵Ac); copper-64 (⁶⁴Cu); copper-67 (⁶⁷Cu); holmium-166 (¹⁶⁶Ho); lead-212 (²¹²Pb); lutetium-177 (¹⁷⁷Lu); radium-223 (²²³Ra); rhenium-186 (¹⁸⁶Re); rhenium-188 (¹⁸⁸Re); samarium-153 (¹⁵³Sm); strontium-89 (⁸⁹Sr); thorium-227 (²²⁷Th) and yttrium-90 (⁹⁰Y). The expression "a radionuclide for radiotherapy" also encompasses combinations of two or more of the above mentioned radionuclides.

As used herein, the expression "radionuclides for PET imaging and radiotherapy" refers to actinium-225 (²²⁵Ac); copper-62 (⁶²Cu); copper-64 (⁶⁴Cu); copper-67 (⁶⁷Cu); gallium-68 (⁶⁸Ga); holmium-166 (¹⁶⁶Ho); lead-212 (²¹²Pb); lutetium-177 (¹⁷⁷Lu); radium-223 (²²³Ra); rhenium-186 (¹⁸⁶Re); rhenium-188 (¹⁸⁸Re); rubidium-82 (⁸²Rb); samarium-153 (¹⁵³Sm); strontium-89 (⁸⁹Sr); thorium-227 (²²⁷Th); yttrium-90 (⁹⁰Y) and zirconium-89 (⁸⁹Zr). The expression "a radionuclide for PET imaging and radiotherapy" also encompasses combinations of two or more of the above mentioned radionuclides.

As used herein, the expression "radionuclides for SPECT imaging and radiotherapy" refers to actinium-225 (²²⁵Ac); copper-64 (⁶⁴Cu); copper-67 (⁶⁷Cu); gallium-67 (⁶⁷Ga); holmium-166 (¹⁶⁶Ho); indium-111 (¹¹¹In); lead-212 (²¹²Pb); lutetium-177 (¹⁷⁷Lu); radium-223 (²²³Ra); rhenium-186 (¹⁸⁶Re); rhenium-188 (¹⁸⁸Re); samarium-153 (¹⁵³Sm); strontium-89 (⁸⁹Sr); technetium-99m (^{99m}Tc³⁺); thallium-201 (²⁰¹TI); thorium-227 (²²⁷Th) and yttrium-90 (⁹⁰Y). The expression "a radionuclide for SPECT imaging and radiotherapy" also encompasses combinations of two or more of the above mentioned radionuclides.

The term "oxysilane" as used herein refers to any organic compounds with one or more oxygen atoms attached to the silicon atom. Non-limiting examples thereof are:

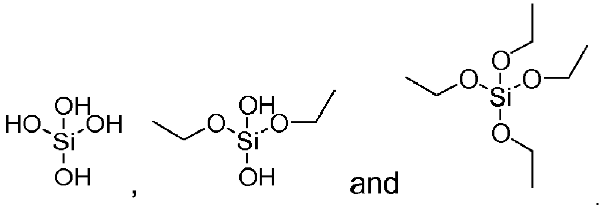

The term "organosilane" as used herein refers to organic compounds containing one or more carbon-silicon bond(s).

The terms "hydrocarbon" and "hydrocarbon chain" are used herein to denote an organic residue consisting of hydrogen and carbon. The hydrocarbon may be fully saturated or it may comprise one or more unsaturations. The hydrocarbon in accordance with the present disclosure may contain any number of carbon atoms between 1 and 50.

The term "alkyl" as used herein refers to a straight or branched hydrocarbon chain fully saturated (no double or triple bonds) hydrocarbon group. The alkyl group may in the present text have 1-15 carbon atoms. Typical alkyl groups include, but are in no way limited to, methyl, ethyl, propyl, isopropyl, butyl, isobutyl, tertiary butyl, pentyl, hexyl and the like.

The term "lower alkyl" as used herein refers to an alkyl having 1-8 carbon atoms.

The term "lower alcohol" as used herein refers to an alcohol having 1-8 carbon atoms.

Numerical ranges: Whenever it is used herein, unless otherwise stated, a numerical range such as "1 to 8" or "1-8" refer to each integer in the given range; e.g., "1 to 8 carbon atoms" and "1-8 carbon atoms" mean that the alkyl group may consist of 1 carbon atom, 2 carbon atoms, 3 carbon atoms, etc., up to and including 8 carbon atoms. There are, however some exceptions which are clear to the skilled persons. In particular, whenever a range is given herein for a molar ratio, such as the Si/P molar ratio in the nanostructures, for a diameter or size, for a pH, for a period of time, for a concentration, for an osmolality or for a temperature, the range includes also all decimal numbers falling within the range, including the upper and lower limits.

As used herein, the term "alkoxy" refers to the formula -OR wherein R is a lower alkyl, e.g. methoxy, ethoxy, n-propoxy, 1 -methyl ethoxy (isopropoxy), n-butoxy, iso-butoxy, sec-butoxy, tert-butoxy, amyloxy, iso-amyloxy and the like. An alkoxy group in accordance with the present disclosure may be optionally substituted.

### BRIEF DESCRIPTION OF THE DRAWINGS

By way of example, embodiments of the present teaching will now be described with reference to the accompanying drawings, in which:
Fig. 1 is an explanatory illustration of a globular shape.
Fig. 2 is an illustration of the structure of the nanostructures showing substructures of the nanostructures
Fig. 3 shows a 1H-NMR spectra of core nanostructures and primed nanostructures
Fig. 4 shows a 31P-NMR spectrum core nanostructures.
Fig. 5 shows a 1H-NMR of coated nanostructures.
Fig. 6 is an illustration of the chemical structure of a section of a nanostructure
Fig. 7 shows the tumor growth curve for treated and untreated groups of tumor bearing mice.
Fig. 8 shows a process scheme illustrating the steps of producing nanostructures.

### DETAILED DESCRIPTION

The present disclosure relates to globular polymeric nanostructures suitable for the use as carriers of radioisotopes (also referred to as radionuclides) for imaging and the treatment of cancer.

The rationale for nanosized materials being suitable as tumor-targeting radiation carriers is related to the enhanced permeation and retention (EPR) effect. The EPR effect is based on the fact that whereas the capillaries of healthy tissues are virtually impermeable to molecules larger than 3-4 nm, capillaries of fast-growing tumor tissue are much leakier. In addition, solid tumors tend to lack functional lymphatics. Combined, these features limit the removal of extravasated nanomaterials from most solid tumors. Because EPR-mediated drug targeting exclusively relies on the pathological properties of the target tissue, that is, enhanced leakiness and poor lymphatic drainage, it is generally referred to as passive tumor targeting.

Although in no way certain or limiting, it is conceivable that the EPR effect often is the basis of the favorable tumor delivery properties of the nanostructures according to the present disclosure.

Notably, the nanostructures according to the present disclosure are above the threshold where they would be excreted through the kidneys and hence either cause damage and/or be lost from the body while, at the same time, being small enough (below 100 nm diameter) to be able to leak out through defective capillaries and diffuse through the intracellular matrix and deliver the radioactivity to the tumor cells. The nanostructures according to the present disclosure have sizes and surface properties making them suitable for targeting of tumors through the EPR effect.

The nanostructures are bioinert, as biodegradability would cause undesirable and uncontrolled loss of e.g. the radioactive isotope from the nanostructure and hence cause radiation damage in important organs.

### Nanostructures

The nanostructures have hydrodynamic diameters between 20 and 50 nm, are strong chelators of multivalent metal ions, and are highly biocompatible.

The nanostructures are strong chelators of multivalent cations. They are therefore less suitable as carriers of neutral or anionic radionuclides.

The nanostructures are polymeric and comprise a highly cross-linked siloxane network.

The nanostructures comprise three substructures - a chelating core, an anchoring layer, and a coating. The core (also referred to as core nanostructure or central part) comprises chelating bisphosphonate groups. The anchoring layer joins the core to the coating layer. The coating layer protects the nanostructures from aggregation and renders the nanostructures highly biocompatible. The coating layer comprises polyethylene glycol joined to the anchoring layer through siloxane bonds.

The present disclosure relates to a plurality of globular nanostructures having an average hydrodynamic diameter between 20 to 50 nm, wherein each nanostructure is a polymeric nanostructure comprising:
a central part comprising monomer residues according to Formula (I): 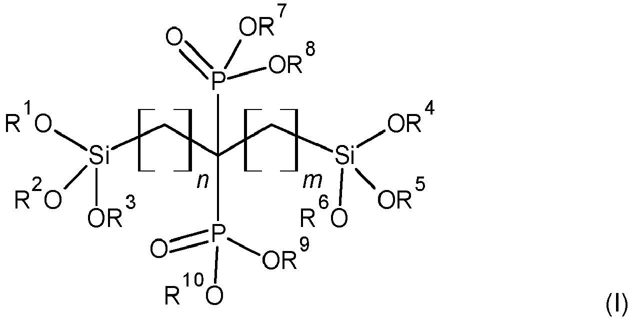 wherein
   - each R¹, R², R³, R⁴, R⁵, and R⁶ is independently chosen from the group consisting of H, a negative charge, and a covalent bond;
   - each R⁷, R⁸, R⁹, and R¹⁰ is independently chosen from the group consisting of H, a negative charge, and lower alkyls, wherein at least 95 % of all of the R⁷, R⁸, R⁹, and R¹⁰ groups are H or a negative charge;
   - n is an integer between 2 and 5; and
   - m is an integer between 2 and 5;
an anchoring layer surrounding the central part, wherein the anchoring layer comprises monomer residues according to Formula (II): 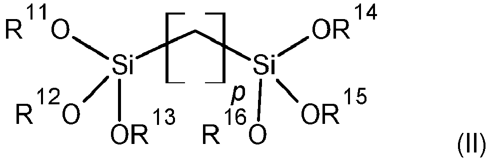 wherein
   - each R¹¹, R¹², R¹³, R¹⁴, R¹⁵, and R¹⁶ is independently chosen from the group consisting of H, a negative charge, and a covalent bond; and
   - p is an integer between 1 and 3;
   and
a coating layer surrounding the anchoring layer, wherein the coating layer comprises monomer residues according to Formula (III): 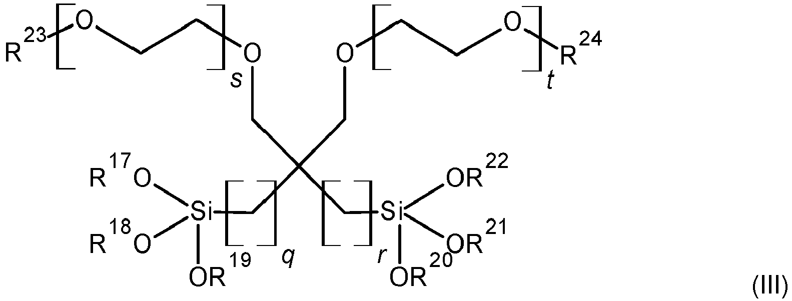 wherein
   - each R¹⁷, R¹⁸, R¹⁹, R²⁰, R²¹, and R²² is independently chosen from the group consisting of H, a negative charge, and a covalent bond;
   - q is an integer between 2 and 5;
   - r is an integer between 2 and 5;
   - each R²³ and R²⁴ is independently chosen from the group consisting of H and lower alkyls;
   - s is an integer between 30 and 105; and
   - t is an integer between 30 and 105.

The different parts of the nanostructures are described in detail below.

The nanostructures are usually prepared and/or used as a plurality of nanostructures, typically but not limiting, as a solution. Compositions comprising nanostructures of the present disclosure will always contain a plurality of the nanostructures. The nanostructures can be characterized by statistical measures, such as, but not limited to, average diameter, average molecular weight, dispersity, density, concentration or size measures such as the percentage passing through certain calibrated filters with nominal cut-off values for the molecular weight.

The nanostructures of the present disclosure have hydrodynamic diameters between 20 and 50 nm. Nanostructures of such a size are suitable for targeting of tumors through the EPR effect.

Preferably, the nanostructures have hydrodynamic diameters between 21 and 40 nm.

It is advantageous to use nanostructures having hydrodynamic diameters between 22 and 37 nm, as this results in strong accumulation of the nanostructures in tumor tissue (see Example 12).

Thus, the hydrodynamic diameter of the nanostructures may preferably between 23 and 35 nm, such as between 25 and 30 nm.

The nanostructures are present as a plurality of nanostructures differing somewhat in size and structure. When a single size of a plurality of nanostructures is given, it should be understood as some measure of the center of the size distribution, most often the mean size. When size is given as a range it should be understood as that said measure of the center of the size distribution falls within the range, and that some portion of the size distribution of the plurality of nanostructures can be bigger or smaller than the range. The width of the size distribution of the nanostructures can be described by the dispersity.

For many applications, it is desirable to have a size distribution as narrow as possible. The nanostructures of the present disclosure have the advantage that they have a fairly narrow size distribution. Typically, the dispersity is low, such as below 1.6, such as between 1 and 1.6, or between 1 and 1.4, or between 1.1 and 1.3, or between 1.13 and 1.27 as measured by DLS and the definition given above.

Thus, in one embodiment, the dispersity is below 1.4.

In another embodiment, the dispersity of the nanostructures is below 1.3.

In a further embodiment, the dispersity of the nanostructures is below 1.2.

The nanostructures of the current disclosure are polymeric and comprise cross-linked polymer networks. The polymer network comprises monomer residues that can form up to 6 bonds to other monomer residues. The majority of the monomer residues in the polymer network comprises 2 silicon atoms, each silicon atom capable of forming 3 siloxane bonds to other silicon atoms.

The polymeric network comprises siloxane bonds between monomer residues.

The polymer network of the nanostructures is highly branched and densely cross-linked, forming a dense three-dimensional network.

Each monomer residue in the nanostructures according to the present disclosure is capable of forming up to six neighboring monomer residues. It is clear to one skilled in the art that realization of complete cross-linking, i.e. that each monomer residue is bound to six neighboring monomer residues, is essentially impossible. Instead, some monomer residues will bear some silanol groups not forming bonds to other silicon atoms.

The average number of bonds to other monomer residues per monomer residue has been found to be a useful measure of the degree of cross-linking in the nanostructure. Typically, the nanostructures of the present disclosure have more than 3 bonds to other monomer residues per monomer residue.

The average number of bonds to other monomer residues per monomer residue can be estimated from the elemental composition of the nanostructure, as formation of a siloxane bond from two silanols is formally a condensation reaction releasing a molecule of water: 2Si-OH → Si-O-Si + H₂O.

In Example 7 is shown an example of such a determination of the average number of bonds to other monomer residues per monomer residue from the elemental composition of the nanostructure, showing that the average number of bonds other monomer residues per monomer residue is above 4, corresponding to 68% of the theoretical maximum of 6 bonds per monomer residue.

It has been found that synthesis conditions that result in highly cross-linked nanostructures also result in nanostructures that are good chelators of metal ions.

As mentioned above, the nanostructures comprise three substructures. The polymer network of each substructure comprises monomer residues of a different structure. The central part, or the core, comprises monomer resides that bear chelating groups. The central part is surrounded by an anchoring layer. The anchoring layer is surrounded by a coating layer. The coating layer comprises polyethylene glycol. The different substructures are described in detail below.

In Fig. 2C is shown a schematic representation of the structure of the nanostructures.

The thickness of the anchoring layer, calculated as half the difference in hydrodynamic diameter between the core nanostructure and the primed nanostructure, (D_{B}-D_{A})/2 in Fig. 2B, is preferably between 0.6 and 5 nm.

The thickness of the coating layer, calculated as half the difference in hydrodynamic diameter between the primed nanostructure and the coated nanostructure, (D_{C}-D_{B})/2 in Fig. 2C, is preferably between 1 and 15 nm.

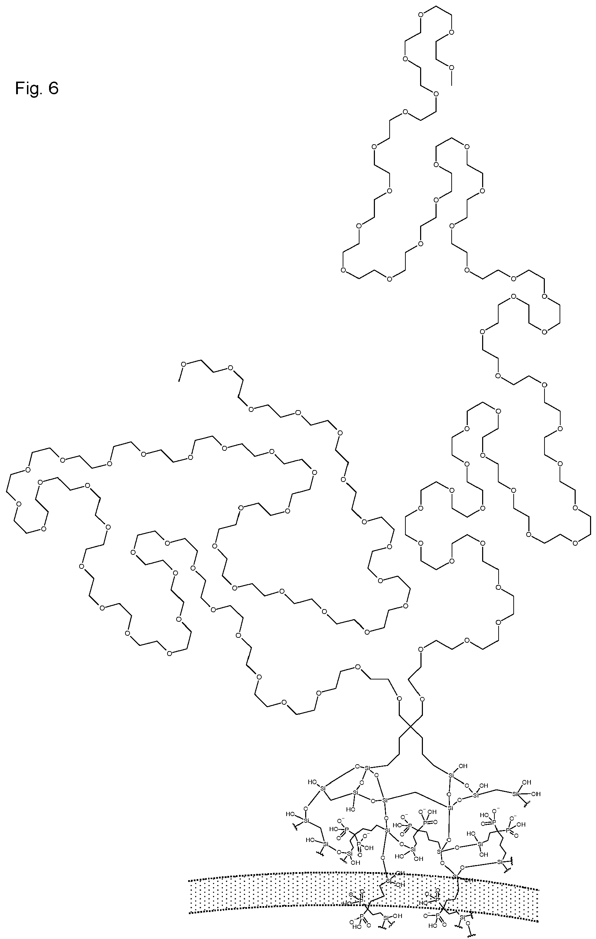

In Fig. 6 is shown a schematic illustration of the molecular structure of a part of a nanostructure according to the present disclosure. The shaded part represents the anchoring layer, the area above the shaded part represents the coating layer and the area below the shaded part represents the central part of the nanostructure. The chemical structures and the substructures of the nanostructure in Fig. 6 are not necessarily drawn to scale.

The central part enables the nanostructure to strongly bind radionuclides.

The anchoring layer binds the coating strongly to the nanostructure.

The coating layer protects the nanostructure from aggregation and makes the nanostructures highly soluble in water. The coating layer also renders the nanostructures highly biocompatible and enables the nanostructures to be long circulating *in vivo.*

The monomer residues of the core, of the anchoring layer, and of the coating layer are bound to each other and to monomer residues of the other substructures through siloxane, Si-O-Si, bonds.

At the interface between the substructures of the nanostructure, the monomer residues of one substructure bind to monomer residues of another substructure through siloxane bonds. The interface between the substructures may comprise a gradual intermixing of monomer residues of the two substructures, rather than a sharp change from one kind of monomer residues to another.

The individual sizes of the substructures are often hard to measure. Often it is useful to use the sizes of nanostructure intermediates used in the synthesis of a nanostructure as approximations of the sizes of the substructures derived from the intermediate.

It is less useful to describe size by degree of polymerization by the molecular weight rather than hydrodynamic diameter. However, this is another way of conceptualizing the structures. The degree of polymerization is included not as limiting, but rather as a reference. For example, for a nanostructure with a core diameter of 17.5 nm derived from 7-bis-(triethoxysilyl)-4,4-bis-(dimethyl-phosphonato)-heptane through a hydrolytic condensation polymerization, with an estimated nanostructure core density of 1.2 g/ml, the estimated degree of polymerization of the central part would be about 5200 monomers and the molecular weight about 2 MDa as measured by DLS and then calculated from the volume and density.

The relative amounts of the different monomer residues present in the nanostructure can be expressed as molar fractions, that is, the number of a specific kind of monomer residues in a nanostructure or in a plurality of nanostructures divided by the total number of monomer residues in the nanostructure or in the plurality of nanostructures.

The molar fractions of the various monomer residues can be analyzed by a number of methods known in the art.

One method to estimate the molar fractions of the monomer residues is a combination of hydrolytic depolymerization with mass spectroscopic analysis. According to this method, samples of nanostructures are digested in alkaline media, resulting in hydrolysis of the siloxane bonds in the polymeric network and a solution of hydrolyzed monomer residues and/or hydrolyzed oligomers of monomer residues. The hydrolyzed monomer residues and/or hydrolyzed oligomers of monomer residues are identified by e.g. LC-MS analysis, and relative amounts calibrated through comparisons to hydrolyzed solutions of the monomers.

Alternatively, monomer residues can be identified through a combination of hydrolytic depolymerization with mass spectroscopic analysis, and relative amounts found through elemental analysis.

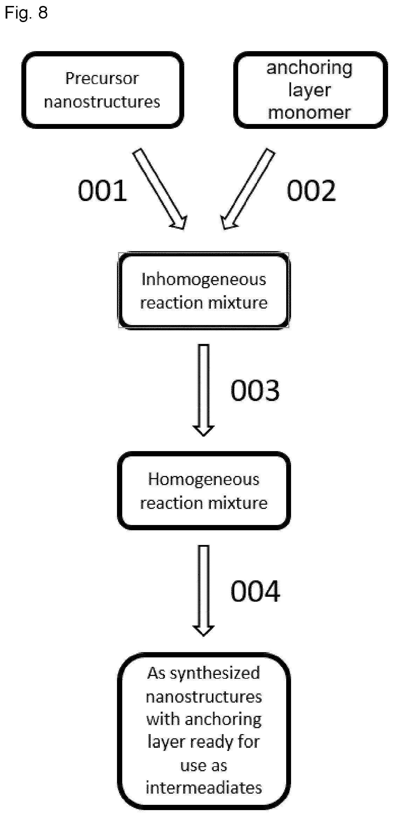

Nanostructures according to the present disclosure can be synthesized by a bottom-up process, as illustrated in Fig. 8. In such a bottom up process the central part, 2A in Fig. 2, is first formed in the absence of the monomers forming the anchoring layer or the coating layer, forming core nanostructures. The anchoring layer is applied to the core nanostructures in the absence of the monomers of the coating layer, forming primed nanostructures, 2B in Fig. 2. The coating layer is applied to the primed nanostructures, forming coated nanostructures, 2C in Fig. 2.

Formation of the central part, the anchoring layer and the coating layer can be realized through hydrolytic condensation polymerization of precursors comprising -Si(OR)₃ groups, where R is lower alkyl. Especially, precursors bearing triethoxysilyl groups are suitable for the synthesis of nanostructures according to the present disclosure.

Nanostructures according to the present disclosure are preferably synthesized using core nanostructures with hydrodynamic diameters between 13 and 35 nm. Synthesis of the nanostructures are described in more detail below.

### Central part

The central part of the nanostructures comprises monomer residues that bear geminal bisphosphonates and that comprise 2 silicon-carbon bonds, henceforth called core monomer residues. The core monomer residues are bound to each other and to the anchoring layer monomer residues through siloxane, Si-O-Si, bonds.

The central part of the nanostructures comprises monomer residues according to Formula (I): 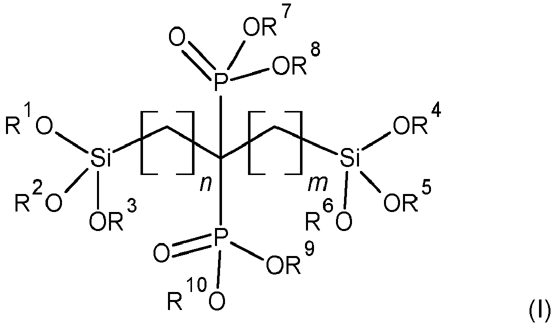 wherein
- each R¹, R², R³, R⁴, R⁵, and R⁶ is independently chosen from the group consisting of H, a negative charge, and a covalent bond;
- each R⁷, R⁸, R⁹, and R¹⁰ is independently chosen from the group consisting of H, a negative charge, and lower alkyls, wherein at least 95 % of all of the R⁷, R⁸, R⁹, and R¹⁰ groups are H or a negative charge;
- n is an integer between 2 and 5; and
- m is an integer between 2 and 5;

The covalent bonds are covalent bonds to the rest of the polymeric structure.

In one embodiment, n and m are independently 2 or 3. Preferably, n and m are both 3.

In one embodiment, the molar fraction of the core monomer residues in the nanostructure is between 5% and 20%.

In another embodiment, the molar fraction of the core monomer residues in the nanostructure is between 20% and 40%.

In a further embodiment, the molar fraction of the core monomer residues in the nanostructure is above 40%.

In a specific embodiment, the monomer residues of the central part are derived from 1,7-bis-(triethoxysilyl)-4,4-bis-(dimethylphosphonato)-heptane through a hydrolytic condensation polymerization.

It is advantageous to synthesis the central part using monomers of high purity, especially with respect to impurities that bear reactive siloxane groups. The use of such monomers of high purity results in a central part comprising to a large fraction, such as above 90%, of monomer according to Formula (I).

The molar fraction of the core monomer residue in the nanostructures is typically between 5% and 58%.

For good binding of radionuclides to the nanostructure, it is advantageous that a high fraction of all R⁷, R⁸, R⁹ and R¹⁰ present in the nanostructure are H or negative charge. Thus, more than 95%, of all R⁷, R⁸, R⁹ and R¹⁰ present in the nanostructure are H or negative charge.

In one embodiment, less than 10%, such as less than 5%, of all R⁷, R⁸, R⁹ and R¹⁰ present in the nanostructure are alkyl.

In addition to the monomer residues according to Formula (I) above, the core may comprise some fraction of other monomer residues. These may be derived from impurities present in the monomer used to synthesize the core. They may also be monomer residues derived from a monomer that is purposefully included in the synthesis of the nanostructure core.

Impurities present in the monomer used to synthesize the core may comprise monomers similar to the monomers resulting in monomer residues according to Formula (I), but comprising only a single silicon. Such impurities may be generated during the synthesis of the core monomer. Other impurities present may include hydrosilylation reagents and degradation and disproportionation products of hydrosilylation reagents, such as HSi(OR)₃, Si(OR)₄, H₂Si(OR)₂, or (RO)₃Si -O-Si(OR)₃ where R is defined as for R¹, R², R³, R⁴, R⁵, and R⁶ above.

Monomer residues derived from a monomer that is purposefully included in the synthesis of the nanostructure core may for example include silylated dyes.

The core may also comprise some amount of reaction solvent irreversibly or essentially irreversibly incorporated into the core, especially if a polyol is used as a reaction solvent for the synthesis of the core. When ethylene glycol is used as a reaction solvent for the synthesis or the core, up to a few percent, w/w (weight/weight), of ethylene glycol may be irreversibly incorporated into the core.

### Anchoring layer

Nanostructures of the present disclosure comprise an anchoring layer, which offers improved binding between inner and outer part of the nanostructure compared to nanostructures not having an anchoring layer.

The anchoring layer of the nanostructures comprises monomer residues that comprise 2 silicon atoms connected through a hydrocarbon linker, henceforth called anchoring layer monomer residues. In the anchoring layer monomer residues, the two silicon atoms are separated by 1 to 3 carbon atoms.

The anchoring layer monomer residues are bound to each other, and to the monomer residues of the central part of the nanostructure and to the monomer residues of the coating layer through siloxane bonds.

The anchoring layer surrounds the central part (the core) of the nanostructures and comprises monomer residues according to Formula (II): 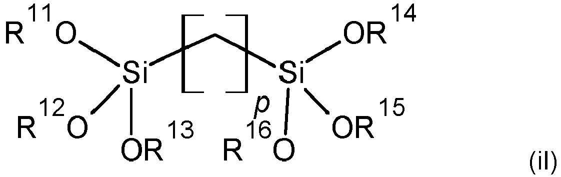 wherein
- each R¹¹, R¹², R¹³, R¹⁴, R¹⁵, and R¹⁶ is independently chosen from the group consisting of H, a negative charge, and a covalent bond; and
- p is an integer between 1 and 3;

The covalent bonds are covalent bonds to the rest of the polymeric structure.

In one embodiment, p is 1 or 2. Preferably, p is 1.

The molar fraction of the core monomer residue in the nanostructures is typically between 39% and 93%.

In one embodiment, the molar fraction of the anchoring layer monomer residues in the nanostructure is between 39% and 60%.

In another embodiment, the molar fraction of the anchoring layer monomer residues in the nanostructure is between 60% and 80%.

In a further embodiment, the molar fraction of the anchoring layer monomer residues in the nanostructure is above 80%.

In a specific embodiment, p is 1 and the molar fraction of the anchoring layer monomer residues in the nanostructure is between 60% and 80%.

In a specific embodiment, the monomer residues of the anchoring layer are derived from bis-(triethoxysilyl)-methane through a hydrolytic condensation polymerization.

Similar to the core, the anchoring layer may comprise some fraction of monomer residues other than monomer residues according to Formula (II). Such monomer residues may comprise monomer residues comprising only one silicon, such as Si(OR)₄ or MeSi(OR)₃.

### Coating layer

The coating layer comprises monomer residues that bear 2 polyethylene glycol groups and 2 silicon atoms connected through a hydrocarbon linker, henceforth called coating monomer residues.

The coating monomer residues are bound to each other and to the anchoring layer monomer residues through siloxane bonds.

The coating monomer residues comprise polyethylene glycol groups. As is known to one skilled in the art, polymers synthesized by the vast majority of methods are not identical chemical entities, but rather comprise a plurality of polymer molecules with different degree of polymerization. The degree of polymerization can be expressed as the number of monomer residues in the polymer, or alternatively be expressed as chain length or by the molecular weight of the polymer. The polyethylene glycol groups of the present disclosure are, unless otherwise specified, present as mixtures of polyethylene glycol groups with different degrees of polymerization.

The coating layer surrounds the anchoring layer and comprises monomer residues according to Formula (III): 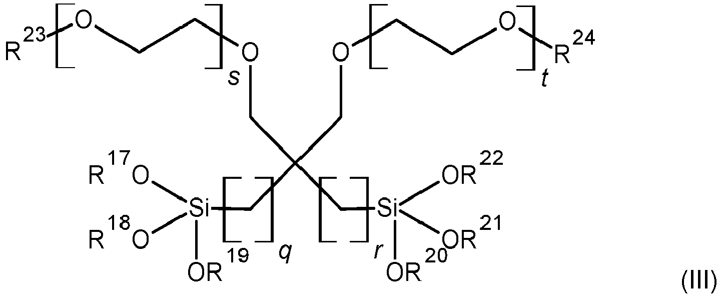 wherein
- each R¹⁷, R¹⁸, R¹⁹, R²⁰, R²¹, and R²² is independently chosen from the group consisting of H, a negative charge, and a covalent bond;
- q is an integer between 2 and 5;
- r is an integer between 2 and 5;
- each R²³ and R²⁴ is independently chosen from the group consisting of H and lower alkyls;
- s is an integer between 30 and 105; and
- t is an integer between 30 and 105.

The covalent bonds are covalent bonds to the rest of the polymeric structure.

Preferably, q and r are both 3.

Preferably, s and t are both between 35 and 55, or both between 45 and 65, or both between 60 and 80.

Preferably, R²³ and R²⁴ and are both methyl or ethyl.

In one embodiment, q and r are both 3, s and t are both between 35 and 55, and R²³ and R²⁴ and are both methyl.

The molar fraction of the coating monomer residue in the nanostructures is typically between 1% and 4.5%.

In a specific embodiment, s and t are both between 35 and 55, R²³ and R²⁴ and are both methyl, and the molar fraction of the coating monomer residue in the nanostructures is between 2% and 3.5%.

In a specific embodiment, the monomer residues of the coating layer are derived from 1,7-bis(triethoxysilyl)-4,4-bis(ω-methyl-(ethyleneoxy)45-methyl)-heptane through a hydrolytic condensation polymerization.

As for the core and the anchoring layer, the coating layer may comprise a fraction of monomer residues other monomer residues according to Formula (III). Impurities present in the monomer used to synthesize the coating layer may comprise monomers similar to the monomers resulting in monomer residues according to Formula (III), but comprising only a single silicon, or comprising only a single polyethylene glycol chain.

### Other components of the nanostructure

As all substructures of the nanostructure may comprise monomer residues other than the monomer residues according to Formulas (I), (II), and (III), the sum of the molar fractions of the monomer residues of the core, the anchoring layer, and the coating layer may be less than 1. This may be due to the use of impure monomer staring materials or due to the purposeful inclusion of monomers other than the ones resulting in monomer residues according to Formulas (I), (II), and (III).

Impurities relating to hydrosilylation processes may not be assignable to a specific substructure, as the monomers of the core, of the anchoring layer, and of the coating layer may all comprise similar or identical impurities introduced through hydrosilylation processes used to synthesize the monomers.

### Specific embodiments of the nanostructures

In a specific embodiment, the molar fraction of the core monomer residues according to Formula (I) in the nanostructure is between 20% and 40% and the molar fraction of the anchoring layer monomer residues according to Formula (II) in the nanostructure is between 60% and 80%.

In a specific embodiment, the molar fraction of the core monomer residues according to Formula (I) in the nanostructure is between 20% and 40%, and the molar fraction of the coating monomer residues according to Formula (III) in the nanostructures is between 2% and 3.5%.

In another specific embodiment, at least 70%, such as at least 80%, such as at least 90%, such as at least 95%, of the monomers in the central part (the core) are monomer residues according to Formula (I).

In another specific embodiment, at least 70%, such as at least 80%, such as at least 90%, such as at least 95%, of the monomer residues in the anchoring layer are monomer residues according to Formula (II).

In another specific embodiment, at least 70%, such as at least 80%, such as at least 90%, such as at least 95%, of the monomer residues in the coating layer are monomer residues according to Formula (III).

In another specific embodiment, at least 70% of the monomers in the central part (the core) are monomer residues according to Formula (I), at least 70% of the monomer residues in the anchoring layer are monomer residues according to Formula (II), and at least 70% of the monomer residues in the coating layer are monomer residues according to Formula (III).

In one embodiment, the average number of bonds to other monomer residues per monomer residue is between 3 and 5.

In another embodiment, the average number of bonds to other monomer residues per monomer residue is above 3.5, such as above 4, such as above 4.5.

In a specific embodiment, the monomer residues of the central part are derived from 1,7-bis-(triethoxysilyl)-4,4-bis-(dimethylphosphonato)-heptane through a hydrolytic condensation polymerization, the monomer residues of the anchoring layer are derived from bis-(triethoxysilyl)-methane through a hydrolytic condensation polymerization, and the monomer residues of the coating layer are derived from 1,7-bis(triethoxysilyl)-4,4-bis(ω-methyl-(ethyleneoxy)₄₅-methyl)-heptane through a hydrolytic condensation polymerization.

### Chelation

The nanostructures according to the present disclosure are strong chelators of multivalent metal ions. This renders the nanostructures suitable as carriers for radionuclides for medicinal purposes, such as for imaging or for the treatment of cancer. To minimize unwanted off-target accumulation of radionuclides *in vivo* it is important the radionuclide is strongly bound to the carrier.

The nanostructures of the current disclosure have the advantage that essentially all the radionuclide remains bound to the nanostructure *in vivo.* An illustration of this is shown in Example 16.

A hundred or more chelating groups in the form of geminal bisphosphonate groups are distributed in the central part of the nanostructure. The chelating groups may be regarded as randomly distributed through the central part and rely on chance to arrange themselves in a way that allows favorable chelation of a metal ion by multiple chelation groups. When relying on chance, it is necessary to incorporate a large excess of chelating groups in the central part to get a reasonable probability of forming a cluster of chelating groups with strong binding affinity.

Phosphonate groups in ester form, that is when R⁷, R⁸, R⁹ and R¹⁰ in the monomer residue according to Formula (I) is alkyl, generally bind metal ions less strongly than phosphonate groups in acid or ionized form, i.e. when R⁷, R⁸, R⁹ and R¹⁰ in the monomer residue according to Formula (I) is H or negative charge, respectively. Therefore, for strong binding of radioisotopes to the nanostructures it is advantageous that a large fraction, such as above 90%, or above 95%, of all of the R⁷, R⁸, R⁹ and R¹⁰ groups present in the nanostructure are H or a negative charge.

In the synthesis of nanostructures, an intermediate where R⁷, R⁸, R⁹ and R¹⁰ are alkyl is used. It has been found that reaction conditions that result in highly cross-linked nanostructures also result in nanostructures where most of the phosphonate groups have been hydrolyzed, i.e. the R⁷, R⁸, R⁹ and R¹⁰ groups present in the nanostructure are H or a negative charge.

In Example 8 is shown NMR data supporting that the phosphonates are essentially completely hydrolyzed.

How strongly the nanostructures bind a metal ion is not easily quantified through direct measurement of binding constants between the nanostructure and the metal ion, but is most conveniently determined by competitive chelation. In Examples 9 and 11 it is shown through competitive chelation with the well-known chelator EDTA that nanostructures according to the present disclosure are good chelators of multivalent metal ions. In Example 16 is shown that the nanostructures bind metal ions strongly also *in vivo.*

### Further advantages of nanostructures according to the present disclosure

It is well known in the art that nanostructures are often prone to aggregation. Aggregation can be the source of short shelf-lives for products containing nanostructures, as the aggregated nanostructures often have significantly different properties than the individual nanostructures.

Nanostructures according to the present disclosure have the advantage that they are very resistant to aggregation, even when being subjected to prolonged storage under harsh conditions. In Example 17 is shown that there are no signs of aggregation even when nanostructures according the present disclosure are stored at 60 °C for more than a month.

Nanostructures according to the present disclosure are highly soluble in water. In Example 15 is shown an example of concentration of a sample of nanostructures to approximately 25 % (w/v) (weight/volume) without any signs of aggregation.

High solubility is advantageous for processing of the nanostructures, as it allows for processing of relatively smaller volumes of solutions. This is especially advantageous at large scale, where dilute solutions might become prohibitively voluminous.

Nanostructures according to the present disclosure are biocompatible. In Examples 12, 13, 14 and 16 is shown that the nanostructures can be injected in mammals substantially without any adverse reactions.

For a nanostructure to have time to distribute into tumor tissue, the nanostructure has to be present in the bloodstream for a sufficiently long time. In other words, they have to be stable under physiological conditions for a sufficiently long time to be distributed to the target site.

The nanostructures of the present disclosure are long circulating, that is, samples injected intravenously are present in the blood for a long time. The plasma half-life, t_{½}, of the nanostructures is long enough to allow for significant accumulation of the nanostructures in the tumor of tumor bearing models.

In Examples 12, 13, 14 and 16 are shown that nanostructures have plasma t_{½} on the order of days.

In Example 20 is shown a comparison of nanostructures according to the present disclosure with a reference nanostructure outside the scope of the present disclosure. The nanostructures according to the present disclosure have significantly longer plasma half-life.

For nanostructures to suitable as carriers of radionuclides for the imaging or treatment of cancer, the nanostructures need to accumulate in tumor tissue.

The nanostructures of the present disclosure accumulate in tumor tissue. In Example 12 is shown that several percent of the injected dose, corresponding to 10% to 20% of the injected dose per gram of tumor tissue, accumulates into the tumor of a tumor bearing mouse model.

Compared to nanostructures disclosed in EP2923712A1, the nanostructures of the present disclosure offer several improvements (see e.g. Example 16). Specifically, the nanostructures of the present disclosure have significantly improved pharmacokinetics, with plasma half-lives several times longer than the nanostructures disclosed in EP2923712A1 Furthermore, nanostructures of the present disclosure have significantly better stability and longer shelf-life. Nanostructures of the present disclosure also bind radionuclides stronger than the nanostructures described in EP2923712A1.

### Synthesis of nanostructures according to the present disclosure

Nanostructures of the current disclosure can be synthesized by a sequence of hydrolytic condensation polymerization reactions of monomer comprising two trialkoxysilane groups. Especially, the polymerization of precursors bearing two triethoxysilyl groups has been found to be particularly suitable for the synthesis of the nanostructures.

The synthesis of the nanostructures comprises the steps of synthesizing the central part (also referred to as the core nanostructure) of the nanostructure; adding or grafting the anchoring layer onto the central part, forming primed nanostructures; and adding or grafting the coating layer to the primed nanostructures, forming nanostructures according to the present disclosure.

The nanostructure is preferably synthesized using core nanostructures having an average hydrodynamic diameter between 14 and 25 nm, or between 15 and 21 nm, or between 16 and 19 nm.

The nanostructure is preferably synthesized using primed nanostructures having an anchoring layer having a thickness of between 1 and 4 nm, such as between 1.1 and 3 nm, such as between 1.2 and 2 nm, such as between 1.3 and 1.8 nm.

Preferably, the nanostructure is synthesized using core nanostructures having an average hydrodynamic diameter between 15 and 21 nm, and an anchoring layer having a thickness of between 1.1 and 3 nm.

### Synthesis of the central part

The central part of the nanostructure can be synthesized through the hydrolytic condensation polymerization of core monomers of the structure according to Formula (IV) 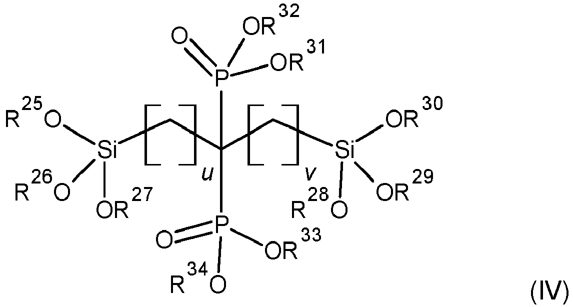 wherein R²⁵ through R³⁴ are each independently chosen to be lower alkyl, where u is an integer between 2 and 5, and v is an integer between 2 and 5.

It is particularly useful to use monomers where R²⁵ through R³⁰ are ethyl, R³¹ through R³⁴ are methyl, and where u and v both are 3.

The synthesis comprises dissolving the core monomer in a mixture of water and a lower alcohol and heating the resulting solution for a period of time depending on the solvent composition and the temperature. Preferably, the solvent is 80% to 90% aqueous ethylene glycol, the reaction temperature is between 120 °C and 150 °C, and the reaction time is between 30 and 400 hours.

Under such reaction conditions, the size of the nanostructures stabilizes after a certain time period to a size dependent on the reaction conditions.

For a given reaction solvent and reaction temperature, the size of the resulting nanostructure can be controlled by the concentration of the monomer in the initial reaction mixture. In Example 4 is shown how the size of the core nanostructure can be varied by varying the concentration of the monomer in the solution.

It is advantageous to use monomers of high purity for the synthesis of the core nanostructures. Especially purity with respect to oligomers appears to be important for the reproducible synthesis of core nanostructures.

In Examples 1A, 2A and 3A is shown examples of different realizations of the synthesis of the central part of the nanostructure.

### Synthesis of the anchoring layer

The anchoring layer is added to the central part of the nanostructure through the reaction of the central part of the nanostructure with anchoring layer monomers according to Formula (V): 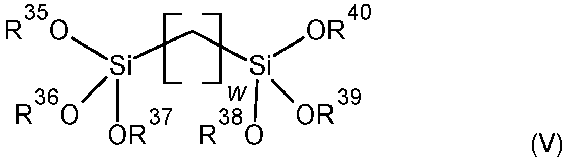 wherein R³⁵ through R⁴⁰ are each independently chosen to be lower alkyl, and w is an integer between 1 and 3.

It is particularly useful to use monomers where R³⁵ through R⁴⁰ are ethyl, and where w is 1.

In Examples 1B, 2B, and 3B are shown different realizations of the addition of the anchoring layer to the central part.

The addition of the anchoring layer may be realized through contacting the core nanostructures with the anchoring layer monomer in the solvent mixture of the synthesis of the core nanostructures at a temperature between 80 °C and 130 °C for between 1 and 24 hours, resulting in a solution of primed nanostructures.

Often the anchoring layer monomer is not fully soluble in the solvent mixture used for the synthesis of the central part. It is convenient to finely disperse the anchoring layer monomer in the reaction medium by vigorous agitation of the reaction mixture at elevated temperature. Under such treatment, the anchoring layer monomer is quickly, such as within a half an hour, such as within 15 minutes, partially hydrolyzed and the partially hydrolyzed anchoring layer monomers dissolve in the reaction medium.

It is often convenient to calculate the amount of anchoring layer monomer as equivalents in relation to the amount of core monomer used to synthesize the solution of core nanostructures.

Typically, between 1 and 10 equivalents of anchoring layer monomer is used.

### Synthesis of the coating layer

The coating layer is added to the primed the nanostructures through the reaction of the primed nanostructure with coating layer monomers according to Formula (VI) 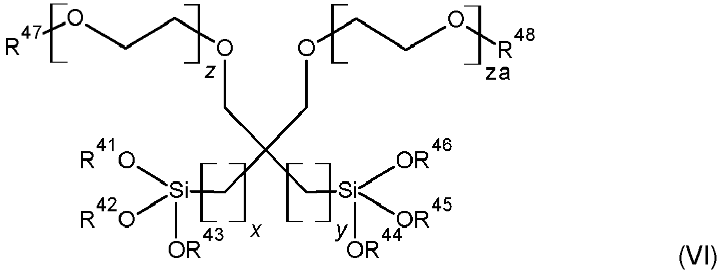 where R⁴¹ through R⁴⁶ are each independently chosen to be lower alkyl, x is an integer between 2 and 5, y is an integer between 2 and 5, z and za are independently from 30 to 105, and R⁴⁷ and R⁴⁸ are each independently chosen from the group consisting of H and lower alkyl.

It has been found especially useful to use monomers where R⁴¹ through R⁴⁶ are ethyl, x and y are both 3, and R⁴⁷ and R⁴⁸ are both methyl.

In Examples 1C, 2C, 3C, 6A, and 6B are shown several examples of the addition of the coating layer to primed core nanostructures.

The addition of the coating layer is most conveniently realized through the addition of the coating layer monomer to a solution of the primed nanostructures in the solvent mixture used to synthesize the primed nanostructures. It is often advantageous to perform the addition of the coating layer at higher dilution than the synthesis of the central part or the synthesis of the primed nanostructures.

It is often advantageous to add the coating layer monomer gradually to the solution of primed nanostructures, such as by additions in portions or by slow infusion of a solution of the coating layer monomer.

The addition of the coating layer is preferably performed at elevated temperatures, such as above 100 °C, such as 110 °C.

The reaction time for the addition of the coating layer is typically between 20 and 100 hours.

It is often convenient to calculate the amount of coating layer monomer as equivalents in relation to the amount of core monomer used to synthesize the solution of primed nanostructures.

Typically, between 0.5 and 5 equivalents of coating layer monomer is used.

The solution at the end of the addition of the coating layer comprises nanostructures as described above, organic solvent and various molecular impurities. For the nanostructures to be useful for use as a carrier of radionuclides for pharmaceutical purposes, the nanostructures need to be present as a solution in water. The reaction solvent can be exchanged for water through ultrafiltration. Using a filtration membrane with a suitable cut-off, such 100 kDa or above, such as 300 kDa or above, such as 1,000 kDa or above, such as 10,000 kDa or above, such as 100,000 kDa or above, such as 1,000,000 kDa or above, allows for the removal of molecular impurities from the solution of the nanostructures concurrently with the solvent exchange.

It has found to be convenient to use tangential flow filtration for the solvent exchange and removal of molecular impurities.

### A specific synthetic route for the synthesis of nanostructures according to the present disclosure

Preferably, the central part (also referred to as the core nanostructure) is synthesized by heating a solution of 1,7-bis(triethoxysilyl)-4,4-bis(dimethyl-phosphonato)heptane in aqueous ethylene glycol; such as by heating a 20 mM to 100 mM solution of 1,7-bis(triethoxysilyl)-4,4-bis(dimethylphosphonato)-heptane in aqueous ethylene glycol for between 40 and 400 hours, such as by heating a 20 mM to 100 mM solution of 1,7-bis(triethoxysilyl)-4,4-bis(dimethyl-phosphonato)heptane in 80% aqueous ethylene glycol to a temperature between 120 °C and 130 °C for between 150 and 400 hours; or such as by heating a 20 mM to 60 mM solution of 1,7-bis(triethoxysilyl)-4,4-bis(dimethyl-phosphonato)heptane in 90% aqueous ethylene glycol to a temperature between 130 °C and 150 °C for between 30 and 100 hours, such as by heating a 30 mM to 40 mM solution of 1,7-bis(triethoxysilyl)-4,4-bis(dimethyl-phosphonato)heptane in 90% aqueous ethylene glycol to a temperature between 135 °C and 145 °C for between 40 and 60 hours.

Preferably, the primed nanostructures are synthesized by heating a solution of core nanostructures formed from 1,7-bis(triethoxysilyl)-4,4-bis(dimethylphosphonato)heptane with between 1 and 5 equivalents of bis(triethoxysilyl)methane, such as by heating a solution of core nanostructures formed from 1,7-bis(triethoxysilyl)-4,4-bis(dimethylphosphonato)heptane with between 2 and 3 equivalents of bis(triethoxysilyl)methane for between 2 and 12 hours at a temperature between 90 °C and 140 °C.

Preferably, the coating layer monomer is 1,7-bis(triethoxysilyl)-4,4-bis(ω-methyl-(ethyleneoxy)₄₅-methyl)-heptane.

When R⁴¹ through R⁴⁶ in Formula (VI) are ethyl, it is convenient that the coating layer monomer is added to a solution of primed nanostructures by a slow infusion of a solution of the coating layer monomer in ethanol.

### Nanostructures comprising radionuclides

A nanostructure according to the present disclosure as described above having one or more radioisotopes bound to the core part of the nanostructure is suitable for use in the imaging and/or in the treatment of cancer. The radionuclide is strongly bound to the nanostructure.

A strongly bound radionuclide is advantageous for pharmaceutical use. A weakly bound radionuclide will to some extent dissociate from the nanostructure and may bind to endogenous compounds. The distribution of the radionuclide in the organism will then not coincide with the distribution of the nanostructures, possibly resulting in more unwanted radiation-related side effects and also less radiation targeting the tumor.

As the coordination chemistry of different isotopes of the same element is the same, some of the examples of realizations of nanostructures with bound radionuclides are given as examples with non-radioactive isotopes of the element in question (also referred to as "cold" isotopes).Nanostructures comprising radioisotopes are conveniently synthesized through mixing of solutions of nanostructures with a solution of a salt of the radionuclide, preferably according to the method described below. If the radiolabeled nanostructure is to be used for pharmacological purposes, the salt of the radionuclide should be a pharmaceutically acceptable salt.

A specific method for the radiolabeling of nanostructures is described below.

In Examples 9, 10 and 11, are shown cold equivalents of nanostructures with loaded Lu, Sm, Y, Gd, Eu, Tb, Zr, Ho, Pr, and Ga.

In Examples 13 and 14 are shown realizations of nanostructures loaded with the radionuclide lutetium-117 (¹⁷⁷Lu).

Typically, a plurality of nanostructures carries on average from 0.01 to 2 radioisotope(s) per nanostructure, such as from 0.5 to 1 radioisotope per nanostructure.

In a specific embodiment, a plurality of nanostructures carries on average from 0.01 to 1 radioisotope per nanostructure.

In another specific embodiment, a plurality of nanostructures carries on average from 0.5 to 2 radioisotopes per nanostructure.

Preferably, the average hydrodynamic diameter of the plurality of nanostructures is between 25 and 35 nm.

The radionuclide may be a radionuclide suitable for imaging, such as PET imaging or SPECT imaging.

The radionuclide may be a radionuclide suitable for radiotherapy and/or imaging.

In one embodiment, the average hydrodynamic diameter of the plurality of nanostructures is between 25 and 35 nm and the nanostructures chelate a radionuclide for PET imaging. The radionuclide for PET imaging may be gallium-68 (⁶⁸Ga).

In another embodiment, the average hydrodynamic diameter of the plurality of nanostructures is between 25 and 35 nm and the nanostructures chelate a radionuclide for SPECT imaging. The radionuclide for SPECT imaging may be technetium-99m in its tri-cationic form (^{99m}Tc³⁺).

In a further embodiment, the average hydrodynamic diameter of the plurality of nanostructures is between 25 and 35 nm and the nanostructures chelate a radionuclide for radiotherapy.

In one embodiment, the average hydrodynamic diameter of the plurality of nanostructures is between 25 and 35 nm and the nanostructures chelate yttrium-90 (⁹⁰Y).

In one embodiment, the average hydrodynamic diameter of the plurality of nanostructures is between 25 and 35 nm and the nanostructures chelate a radionuclide for imaging and/or radiotherapy. The radionuclide for imaging and/or radiotherapy may be lutetium-177 (¹⁷⁷Lu).

In another embodiment, the average hydrodynamic diameter of the plurality of nanostructures is between 25 and 35 nm, the molar fraction of the core monomer residues in the nanostructure is between 20% and 40%, the molar fraction of the anchoring layer monomer residues in the nanostructure is between 60% and 80%, and the molar fraction of the coating monomer residue in the nanostructures is between 2% and 3.5%, and the nanostructure chelates on average from 0.5 to 2 radionuclide ions for imaging and/or radiotherapy, such as lutetium-177 (¹⁷⁷Lu), per particle.

In one embodiment, the average hydrodynamic diameter of the plurality of nanostructures is between 25 and 35 nm and the nanostructure chelates a radionuclide for imaging and/or radiotherapy, such as samarium-153 (¹⁵³Sm).

### Pharmaceutical compositions comprising nanostructures

The present disclosure further relates to a pharmaceutical composition comprising a plurality of globular nanostructures according to the present disclosure, water, and at least one excipient.

The at least one excipient may be an antioxidant or a cryoprotectant. The antioxidant is preferably thioglycerol and/or gentisic acid. The cryoprotectant is preferably glycerol.

One of the antioxidants of choice for acidic injection solutions is sodium metabisulphite. Sodium metabisulphite suppresses the degradation of the nanostructures through oxidation of polyethylene glycol chains in the coating layer. Surprisingly, it has been found that sodium metabisulphite interferes with the binding of metal ions to the nanostructures, making sodium metabisulphite a less suitable excipient for formulations of nanostructures according to the presentdisclosure. In Example 11 is shown a comparison of the metal binding strength of nanostructures with and without the presence of sodium metabisulphite, showing that the presence of sodium metabisulphite more than doubles the amount of the Lu extracted from the nanostructures by competitive chelation with EDTA.

In one embodiment, the pharmaceutical composition comprises an antioxidant. The antioxidant is preferably thioglycerol and/or gentisic acid.

In one embodiment, the pharmaceutical composition comprises an antioxidant other than a sulphite or an ascorbate.

In another embodiment, the pharmaceutical composition comprises a cryoprotectant.

In one embodiment, the pharmaceutical composition comprises an oxidant and a cryoprotectant.

In one embodiment, the pharmaceutical composition comprises thioglycerol.

In another embodiment, the pharmaceutical composition comprises gentisic acid.

In a further embodiment, the pharmaceutical composition comprises glycerol.

In yet another embodiment, the pharmaceutical composition comprises thioglycerol and gentisic acid.

In another embodiment, the pharmaceutical composition comprises thioglycerol, gentisic acid and glycerol.

Thus, the pharmaceutical composition comprises a solution comprising nanoparticles, wherein the nanoparticles are as described above. For simplicity, and clarity, the solution is below referred to as a "carrier formulation".

Depending on the use of the pharmaceutical composition, the nanostructures may be "empty nanoparticles", i.e. they do not carry any chelated ions or be "loaded nanostructures", i.e. carry multivalent metal ions. The multivalent metal ions may be radioisotopes (radionuclides), or may be non-radioactive multivalent metal ions.

### Pharmaceutical composition comprising "empty" nanostructures

In the case when the pharmaceutical comprises "empty" nanoparticles, the carrier formulation is preferably formulated to be suitable for storage and shipment, and for, at a later time point, being combined with a radioisotope to form an injection formulation. The injection formulation is suitable for injection into an organism such as a living human with the purpose of imaging and/or treatment of a pathological condition such as cancer.

The carrier formulation comprises as an acidic solution of nanostructures, water and at least one excipient as described below.

The carrier formulation has a low pH, as required by the method for radiolabeling described below. Preferably, the carrier formulation has a pH between 1.5 and 2.5.

In a preferred embodiment, the formulation comprises thioglycerol, gentisic acid, and glycerol. In such a formulation thioglycerol and gentisic acid function as antioxidants, and glycerol functions as a cryoprotectant. Such a formulation has the advantage that it significantly increases the shelf-life of the nanostructures, allows for the storage of the solution as a frozen solution, and that it does not interfere with radiolabeling of the nanostructures.

Nanostructures according to the present disclosure comprises polyethylene glycol chains. Oxidation of the polyethylene glycol chains in the nanostructures results in the formation of free polyethylene glycol chains in the solution. The presence of such molecular degradation products in the solution can make the solution unsuitable for pharmaceutical use, and the generation of molecular degradation products thus lowers the shelf-life of the nanostructure solution. The formulation of the carrier formulation as described above has the advantage that it significantly increases the shelf-life of the nanostructures through the suppression of the formation of molecular degradation products formed through oxidation of polyethylene glycol chains in the coating layer of the nanostructures. In Example 17 is shown that nanostructure solutions formulated according to the present disclosure have extended shelf-lives.

Nanostructures formulated with thioglycerol and gentisic acid can be radiolabeled with the method described below without increasing the amount of the radionuclide that can be extracted from the nanostructures by competitive chelation with EDTA. Nanostructures formulated with thioglycerol and gentisic acid further do not risk generating calcium oxalate precipitates when administered into a patient together with calcium (see below for a discussion regarding the presence of calcium In pharmaceutical composition used for intravenous administration of the nanonparticles).

The shelf-life of a pharmaceutical composition can often be increased through freezing and storage at low temperature. However, freezing and thawing of solutions of nanostructures according to the present disclosure in pure water results in significant aggregation of the nanostructures, making them unsuitable for pharmaceutical use. The formulation of the carrier formulation according to the present disclosure has the advantage that it allows for the freezing of the solution, thereby significantly increasing the shelf-life of the nanostructures. In Examples 1E, 1F and 18 is shown that nanostructure solutions formulated according to the present disclosure can be frozen and stored as frozen solutions. The formulation of the carrier formulation also has the advantage that it allows for the storage of the frozen solution in a normal freezer, such as at about -20 °C, and that the freezing of the solution does not require flash freezing.

In one embodiment, the pharmaceutical composition comprises an antioxidant.

In one embodiment, the pharmaceutical composition comprises an antioxidant other than a sulphite or an ascorbate.

In another embodiment, the pharmaceutical composition comprises a cryoprotectant.

In one embodiment, the pharmaceutical composition comprises an oxidant and a cryoprotectant.

In one embodiment, the pharmaceutical composition comprises thioglycerol.

In another embodiment, the pharmaceutical composition comprises gentisic acid.

In a further embodiment, the pharmaceutical composition comprises glycerol.

In yet another embodiment, the pharmaceutical composition comprises thioglycerol and gentisic acid.

In another embodiment, the pharmaceutical composition comprises thioglycerol, gentisic acid and glycerol.

In one embodiment, the pharmaceutical composition has a pH below 3.5, such as below 3, such as below 2.5, such as between 1.5 and 2.5.

In a specific embodiment, the pharmaceutical composition has a pH below 3.5 and comprises thioglycerol, gentisic acid, and glycerol.

In another embodiment, the pharmaceutical composition comprises between 0.01 mg/ml and 100 mg/ml thioglycerol, such as between 0.1 mg/ml and 10 mg/ml thioglycerol, such as between 0.2 mg/ml and 2 mg/ml thioglycerol.

In a further embodiment, the pharmaceutical composition comprises between 0.01 mg/ml and 100 mg/ml gentisic acid, such as between 0.03 mg/ml and 3 mg/ml gentisic acid, such as between 0.1 mg/ml and 1 mg/ml gentisic acid.

In another embodiment, the pharmaceutical composition comprises between 1 mg/ml and 500 mg/ml glycerol, such as between 3 mg/ml and 300 mg/ml glycerol, such as between 10 mg/ml and 100 mg/ml glycerol.

In a specific embodiment, the pharmaceutical composition has a pH below 3.5 and comprises between 0.1 mg/ml and 10 mg/ml thioglycerol, between 0.03 mg/ml and 3 mg/ml gentisic acid, and between 3 mg/ml and 300 mg/ml glycerol. Such a pharmaceutical composition comprising "empty" nanostructures can be used as the solution in step a) of the method for radiolabeling nanostructures as described below. Such a pharmaceutical composition comprising "empty" nanostructures can also constitute the aqueous solution of globular nanostructures in the kit described below.

### Pharmaceutical compositions for intravenous administration

Nanostructures according to the present disclosure are strong chelators of calcium. Therefore, injection of the nanostructures without a coadministration of an adequate amount of calcium can adversely disrupt functions in the patient dependent on the concentration of calcium ions in the blood, such as cardiac function. Use of the nanostructures for injection must therefore be accompanied with an injection of calcium. The nanostructures and the calcium may be administered together in a single injection or infusion. One of the antioxidants of choice for acidic injection solutions is ascorbic acid. Ascorbic acid is known to form oxalic acid upon degradation. Oxalic acid is incompatible with calcium in solutions for injection, as calcium oxalate is highly insoluble and prone to form precipitates. This is especially true at neutral or slightly basic pH as encountered in blood, risking the formation of calcium oxalate precipitates in the blood stream of a patient. Ascorbic acid or ascorbate salts are therefore unsuitable for formulations of nanostructures according to the present disclosure. Thus, other antioxidants, e.g. thioglycerol and gentisic acid, are preferably used since these antioxidants do not risk generating calcium oxalate precipitates when administered into a patient together with calcium.

In a specific embodiment, in the pharmaceutical composition the average hydrodynamic diameter of the plurality of nanostructures is between 25 and 35 nm, the molar fraction of the core monomer residues in the nanostructure is between 20% and 40%, the molar fraction of the anchoring layer monomer residues in the nanostructure is between 60% and 80%, and the molar fraction of the coating monomer residue in the nanostructures is between 2% and 3.5%, and the nanostructure chelates on average from 0.5 to 2 radionuclide ions for imaging and/or radiotherapy, such as lutetium-177 (¹⁷⁷Lu), per particle. The pharmaceutical composition is preferably contained in a vial or syringe. The pH of such a pharmaceutical composition is essentially neutral. Such a pharmaceutical composition is suitable for injection.

### Medical applications of nanostructures and compositions comprising nanostructures according the present disclosure

Nanostructures according the present disclosure and pharmaceutical compositions comprising such nanoparticles can be used in different applications as described below.

### Use of the nanostructures in radionuclide therapy or imaging

The present disclosure also relates to use ofthe nanostructures according to the present disclosure in radionuclide therapy or medical imaging. Often the goal of such use is the treatment or imaging of cancer.

Preferably, the nanostructures used for radionuclide therapy or medical imaging are radiolabeled using the method described below and comprised in a pharmaceutical composition as described above. This may be realized through the use of a kit as described below.

When nanostructures according to the present disclosure are used for the treatment of cancer, they may be used in combination with external beam radiotherapy and/or in combination with chemotherapy.

In one embodiment, the radionuclide is a radionuclide for PET imaging and the use is PET imaging.

In another embodiment, the radionuclide is a radionuclide for SPECT imaging and the use is SPECT imaging.

In a further embodiment, the radionuclide is a radionuclide for radiotherapy, and the use is radionuclide therapy.

Different radionuclides are described below and in the section "Definition of terms".

### Use of "cold" nanostructures

Nanostructures without any radionuclide are sometimes known as cold nanostructures, in analogy to nanostructures comprising radionuclides, which may be referred to as hot nanostructures. The cold nanostructures may either not comprising any chelated multivalent metal ions, or may be loaded with a non-radioactive metal ion.

Nanostructures may be loaded with non-radioactive metal ions by a method similar to the method described below, except that the salt of a radionuclide is exchanged for a non-radioactive salt.

As described above, cold "empty" nanostructures may be used as intermediates in the production of a pharmaceutical composition.

Nanostructures according to the present disclosure in the absence of a radionuclide can also be used for pharmaceutical purposes.

Use of nanostructures in the absence of a radionuclide may include the use to induce tolerance to the nanostructures in a patient. Nanostructures according to the present disclosure are covered in polyethylene glycol. It is known that some individuals exhibit an immune response to polyethylene glycol, as shown for example by the presence of polyethylene binding antibodies. Use of nanostructures comprising polyethylene glycol may thus in certain patients be complicated by an immune response to the nanostructures, for example through anaphylactic reactions upon exposure to the nanostructures or through accelerated clearance of the nanostructure from the blood. In such circumstances, treatment of a patient with cold "empty" nanostructures may induce tolerance to the nanostructures in the patient, allowing subsequent pharmaceutical use of hot nanostructures.

Similarly, cold nanostructures may be used to deplete the reservoir of immune system components reactive to the nanostructures prior to administration of hot, i.e. radiolabeled, nanostructures. In such cases, tolerance to the nanostructures is not induced, but rather the ability of the immune system of a patient temporarily exhausted by the cold nanostructures, allowing subsequently administrated hot nanostructures to circulate freely.

Cold "empty" nanostructures according to the present disclosure may also be used as scavengers for multivalent metal ions in a patient. Such use may include scavenging of toxic metal ions. In Example 10 is shown examples of nanostructures according to the present disclosure binding strongly to a variety of multivalent cations, with only trace amounts of unbound metal ions. This indicates that the cold nanostructures can be used for scavenging of essentially any multivalent cation.

Cold nanostructures according to the present disclosure may also be used as a contrast agents. In such cases the nanostructures may be loaded with metal ions that infer additional contrast in an imaging modality, such as gadolinium or manganese for magnetic resonance imaging.

### Method for radiolabeling a plurality of globular nanostructures

The present disclosure also relates to a method for radiolabeling a plurality of globular nanostructures with a multivalent cationic radionuclide. Notably, the same method steps can be used to load nanostructures with non-radioactive multivalent metal ions.

The method is used to load a radioisotope into nanostructures in an optimized way. This serves the purpose of minimizing loading time and maximizing dissociation time. The loading time must be short to make it a practically useful product, especially when the radioisotope is short lived. The dissociation time must be long to avoid radioisotope leakage from the particles after injection into a patient which would cause undesirable radiotoxicity.

The method has the advantage that when nanostructures are loaded with radioisotopes by the method, the radioisotopes are more strongly bound to the nanostructure than when conventional loading is employed. It further has the advantage that it can be performed within a few hours, allowing for both the preparation and use of the radiolabeled nanostructures during the same working day. The method further has the advantage that it can be realized through the use of procedures commonly used in radiopharmacies, as implemented at hospitals.

Chelating phosphonate groups of the kind found in the nanostructures of the current disclosure are acidic, i.e. at high pH they ionize into negatively charged phosphonates. The negatively charged dissociated phosphonate groups are better ligands for metal ions, and it is generally advantageous to perform complexation reactions between phosphonate ligands and metal ions at high pH.

Surprisingly, it has been found that when nanostructures according to the present disclosure are loaded with multivalent metal ions at low pH and the pH is subsequently increased to a neutral pH or close to a neutral pH, such as a physiological pH, the metal ions are more strongly bound to the nanostructures than when the nanostructures are loaded with the metal ions at a more alkaline pH.

The method for radiolabeling a plurality of globular nanostructures with a multivalent cationic radionuclide comprises the steps of:
a) providing a solution having a pH of below 3.5 and comprising a plurality of globular nanostructures not comprising a radionuclide or a multivalent metal ion; and
b) contacting the solution of step a) with a pharmaceutically acceptable salt of the radionuclide; and
c) adjusting the pH of the solution to above 6.

The solution of nanostructures used in step a) preferably has a pH below 3, such as below 2.5. More preferably, the solution has pH between 1.5 and 2.5.

The solution of nanostructures used in step a) is generally an aqueous solution of nanostructures. The solution may further comprise excipients, such as stabilizers, antioxidants, cryoprotectants, preservatives, and antimicrobials.

The pharmaceutically acceptable salt of the radionuclide used in step b) is typically used as a solution of the radioisotope. Contacting the solution of nanostructures with the radioisotope can be realized through the mixing of the solution of the nanostructures and the solution of the radioisotope.

The radionuclide in step b) may be used in an amount from 0.01 to 2 atom(s) of the radionuclide per nanostructure, such as from 0.5 to 1 atom of the radionuclide per nanostructure.

It is advantageous to incubate the solution for some time after step b) prior to performing step c). Preferably, this incubation is performed at slightly elevated temperature, such as between 40 °C and 80 °C, such as between 50 °C and 70 °C, such as between 50 °C and 60 °C. The incubation period is preferably at least 15 minutes, such as between 30 minutes and 3 hours.

The adjustment of pH in step c) may be realized through addition of a buffer solution to the solution of the nanostructures. It is also conceivable to adjust the pH through the addition of a strong base, such as an alkali hydroxide or an alkaline earth metal hydroxide.

It is advantageous to incubate the solution for some time after step c) prior to use of the radiolabeled nanostructures. Preferably, this incubation is performed at slightly elevated temperature, such as between 40 °C and 80 °C, such as between 50 °C and 70 °C, such as between 50 °C and 60 °C. The incubation period is preferably at least 15 minutes, such as between 30 minutes and 3 hours.

The solution obtained after step c) or after incubation after step c) can readily be analyzed for chemical and radiochemical purity by methods known to one skilled in the art.

In Examples 1F and 10 is shown realizations of the method using cold equivalents of some radioisotopes. In example 13 and 14 are shown realizations of the method using some radioisotopes (also referred to as radionuclides).

In Example 9 is shown a comparison of the method described above with a conventional metal loading method, demonstrating that the method of the present disclosure results in stronger binding between the nanostructure and the radioisotope.

In one embodiment, the solution of nanostructures in step a) has a pH between 1.5 and 3, such as between 1.9 and 2.5.

In another embodiment, the radionuclide in step b) is used in an amount of from 0.01 to 1 atom of the radionuclide per nanostructure.

In a further embodiment, the radionuclide in step b) is used in an amount from 0.5 to 2 atom of the radioisotope per nanostructure.

In one embodiment, the radionuclide in step b) is suitable for use in PET imaging.

In another embodiment, the radionuclide in step b) is suitable for use in SPECT imaging.

In a further embodiment, the radionuclide in step b) is suitable for use in radiotherapy.

In a specific embodiment, the radionuclide in step b) is gallium-68 (⁶⁸Ga).

In another specific embodiment, the radionuclide in step b) is technetium-99m in its tri-cationic form (^{99m}Tc³⁺).

In a further specific embodiment, the radionuclide in step b) is lutetium-177 (¹⁷⁷Lu).

In yet another specific embodiment, the radionuclide in step b) is yttrium-90 (⁹⁰Y).

In a further specific embodiment, the radionuclide in step b) is samarium-¹⁵³ (¹⁵³Sm).

In one specific embodiment, the adjustment of pH in step c) is performed by the addition of a buffer comprising Tris, with a pH above 7.5.

In another specific embodiment, the solution is incubated at between 50 °C and 70 °C for between 30 minutes and 2 hours between step b) and step c).

In a further embodiment, the solution is incubated at between 50 °C and 70 °C for between 1 hour and 3 hours after step c) before the radiolabeled nanostructures are used.

### Kit

The present disclosure also relates to a kit, allowing for the realization of radiolabeling of nanostructures according to the present disclosure by the method disclosed above.

The kit can be used in a radiopharmacy to produce one or a small number of doses of radiolabeled nanostructures for use in the imaging and/or treatment of a pathological condition such as cancer.

The kit can be used in combination with a source of radioisotope to generate radiolabeled nanostructures suitable for intravenous use.

The kit comprises an aqueous solution of globular nanostructures according to the present disclosure not comprising a radionuclide or a multivalent metal ion, having a pH below 3.5; an aqueous solution of a pharmaceutically acceptable buffer having a pH of at least 6; and instructions for use, allowing for the realization of the method of radiolabeling as described above.

The kit may optionally comprise a mixing vessel, an apparatus for sterile filtration, a pharmaceutically acceptable dilutant, a centrifugal filter, and/or equipment for the shielding of ionizing radiation.

The kit comprises a volume of an aqueous solution of globular nanostructures as described above and having a pH below 3.5, contained within a suitable container, such as a pharmaceutical glass vial with a rubber cap. The solution has a composition making it suitable for use as the nanostructure solution in step a) of the method for radiolabeling a plurality of globular nanostructures described above. The volume of the formulation is such that the resulting radiolabeled nanostructures are enough for quality control operations and for at least one dose to a patient. Typically, the volume is under 10 ml.

The kit further comprises a volume of an aqueous solution of a pharmaceutically acceptable buffer having a pH of at least 6 contained within a suitable container, such as a pharmaceutical glass vial with a rubber cap. The buffer is of sufficient strength and volume, and has a sufficiently high pH, to be suitable for use for the pH adjustment in step c) of the method described above.

The kit further comprises instructions for use, allowing for the realization of the method of radiolabeling above. The instructions allow adequately trained and/or certified personnel to carry out the method. The instructions preferably also detail the procedure to formulate the radiolabeled nanostructures into the dosage form to be used in a patient. The instructions preferably also detail appropriate quality control measures. The instructions may also include instructions for the handling and disposal of radioactive waste, instructions for the administration of the resulting radiopharmaceutical to a patient, instructions for calculation of doses, and instructions for imaging.

The kit may further comprise a mixing vessel, suitable for the use in steps b) and c) of the method described above. The vessel may be of a size suitable to contain the solution of radiolabeled nanostructures as obtained at the end of the radiolabeling process, or it may be significantly larger, allowing for the dilution of the solution of radiolabeled nanostructures to a significantly larger volume. Such dilution may be advantageous if the radiolabeled nanostructures are to be administrated by slow infusion.

The kit may further comprise an apparatus for sterile filtration, such as syringe filters, syringes and receiving vessels.

The kit may further comprise a pharmaceutically acceptable dilutant, such as physiological saline solution (0.90% w/v of NaCl in water) or a calcium containing buffer.

The kit may further comprise equipment for quality control. Such equipment for quality control may compromise centrifugal filters, allowing for the quantification of the binding of the radioisotope through determination of the activity of the permeate. Such equipment for quality control may also comprise components necessary for the implementation of chromatographic methods, such as TLC plates, eluents, and sample preparation components for analysis by HPLC, suitable for the control of the identity and radiochemical purity of the radiolabeled nanostructures.

The kit may further comprise equipment for the shielding of ionizing radiation. Such equipment may comprise shielded containers, suitable to store and transport vessels for radioactive material in. Such shielded containers may be of a size and shape suitable to enclose containers that are part of the kit. Such equipment for the shielding of ionizing radiation may also include shielded equipment for the handling of liquids, such as shielded syringes.

The kit may also comprise secondary and tertiary enclosures, allowing the collection of the components of the kit into one or a small number of units, suitable for commercial storage and shipment of the kit. Such enclosures may comprise paper and/or plastic boxes, and/or plastic or paper bags. Such enclosures may be suitable for storage of the kit or parts of the kit in a freezer.

The kit may also comprise one or more identifiers, such as one or more bar codes or one or more RFID chips. Such identifiers may be present in or on the kit or in one or more of the vessels or containers included in the kit. An identifier can be used to uniquely identify the kit for purposes of quality control or inventory control.

The kit may contain all the components necessary for the preparation of a pharmaceutical formulation of radiolabeled nanostructures, suitable for injection into a patient, except the radioisotope. It is also possible that components necessary for the preparation of a pharmaceutical formulation of radiolabeled nanostructures that are commonly found in a preparative radiopharmacy are not contained within the kit. If so, the instructions detail the further equipment that is necessary for realization of the radiolabeling.

In one embodiment, the aqueous solution of globular nanostructures comprised in the kit is pharmaceutical composition according to the present disclosure.

In another embodiment, the aqueous solution of globular nanostructures comprised in the kit is a pharmaceutical composition according to the present disclosure, and the aqueous solution of a pharmaceutically acceptable buffer comprises Tris (tris(hydroxymethyl)aminomethane).

In further embodiment, the kit further comprises equipment for quality control comprising equipment and reagents for the preparation of samples for analysis by size exclusion chromatography.

### EXAMPLES

Examples of different embodiments of the present disclosure are described below.

### General experimental details

Materials, reagents and solvents were obtained from commercial sources and were used without further purification unless otherwise noted. Solvents were of reagent grade or similar if not otherwise noted. Reactions were carried out under N₂ unless otherwise noted.

SIR-200, a thiolated polystyrene resin was purchased from ResinTech, USA (resintech.com) and activated by aqueous NaHCO₃, carefully washed in ethanol and toluene, and dried azeotropically before use.

HPLC was performed on a Hewlett Packard Series 1100 equipped with an Agilent Poroshell 120 EC-C18 4.6 × 50 mm column, a DAD detector (a diode-array detector) recording at 220 nm and an ELSD detector (an evaporative light scattering detector) (ELSD settings: 40 °C, 1.4 I N2/min, gain=4, impactor on), using a nonlinear gradient starting at 43% acetonitrile in water, at 1 ml/min with an oven temperature of 40 °C.

DLS was measured using a Malvern Instruments Zetasizer Nano ZEN3600 and processed using the general process setting in the Zetasizer software.

SEC (size-exclusion chromatography) was performed on a Younglin Instrument YL9100 equipped with an Agilent Bio SEC-5 1000 Å column eluting at 1.2 ml/min at ambient temperature, a DAD detector recording at 220 nm, 280 nm and 560 nm, and an ELSD detector (ELSD settings: 60 °C, 1.2 I N₂/min, gain=4).

NMR spectra were recorded on a Varian Unity Inova 400 MHz (₁H at 399.95 MHz, 13C at 125.68 MHz) spectrometer using the residual solvent peak as internal standard for 1H NMR and 13C NMR.

When purities are referenced in the examples below, this refers to chromatographic purities, area/area, either from HPLC-ELSD or SEC-ELSD chromatograms, for molecular compounds and nanostructures, respectively.

Compounds 1,7-bis(triethoxysilyl)-4,4-bis(dimethylphosphonato)heptane, 1-(ω-methyl-(ethyleneoxy)₄₅-methyl)-3,5-bis(3-(triethoxysilyl)propyloxy)-benzene, and 2,2-diallyl-propane-1,3-diol were synthesized according to literature procedures.

### Example 1 - Synthesis of nanostructures

Nanostructures according to the present disclosure were synthesized, filtered and formulated as described below. This example shows a large lab scale synthesis of nanostructures.

### Example 1A - Synthesis of the core nanostructure

A 5-liter jacketed reactor was equipped with a mechanical stirrer, a temperature probe, and a reflux condenser topped with a connection to a vacuum-nitrogen manifold. The reactor was charged with 93.60 g of 1,7-bis(triethoxysilyl)-4,4-bis(dimethylphosphonato)heptane of a purity of 94.5% and 4501 g of 90.0 % (v/v) aqueous ethylene glycol, prepared by mixing 4414.0 g of ethylene glycol with 440.5 g of ultrapure water. The solution was degassed. The mantle temperature was increased to 155 °C over a period of 30 min with stirring, during which the solution became clear. After 1.5 hour at the set mantle temperature, a gentle reflux was obtained and the mantle temperature was adjusted to 151 °C. The reaction mixture was kept at reflux for 47 hours before being cooled down to 20 °C. The nanostructure solution was filtered through double glass fiber filters (GF/A).
Mean diameter of the produced nanostructures: 17.6 nm. Ð_{d} = 1.21. [P](ICP-OES) = 69 mM, [Si](ICP-OES) = 72 mM.

### Example 1B - Addition of the anchoring layer

A 5-liter jacketed reactor equipped with a mechanical stirrer, a temperature probe, and a connection to a N₂/vacuum manifold was charged with 4338 g of the solution of precursor nanostructures from Example 1A. The mantle temperature was set to 100 °C and when the inner temperature was above 99 °C, 116.48 g of bis(triethoxysilyl)methane was added. The mixture was stirred at 300 RPM for 5 minutes, after which the mixture was homogenous and stirring speed lowered to 150 RPM. Heating was continued for 4 h before the solution was cooled to ambient temperature and filtered through double glass fiber filters.
Average diameter (DLS) = 19.7 nm, Ð_{d} = 1.20, [P](ICP-OES) = 64 mM, [Si](ICP-OES) = 225 mM.

### Example 1C - Addition of the coating layer

A 10-liter jacketed reactor equipped with a mechanical stirrer, a temperature probe, an addition funnel heated to 40 °C by a heating fan, and reflux condenser topped with a connection to a N₂/vacuum manifold was charged with 6330 g of 90 % aqueous ethylene glycol and 3902 g of the solution of primed nanostructures from Example 1B. The jacket temperature was set to 125 °C for 1 hour before being lowered to 110 °C. A coating solution was prepared by dissolving 548.2 g of 1,7-bis(triethoxysilyl)-4,4-bis(ω-methyl-(ethyleneoxy)₄₅-methyl)-heptane (prepared as described in Example 22) in 548.2 g of anhydrous ethanol under heating to 40 °C. When the inner temperature in the reactor reached 105 °C, 250 ml of the coating solution was added. The remainder of the coating solution was added slowly via the addition funnel at 50 ml/h. The jacket temperature was kept at 110 °C until 48 hours after the initial addition of coating solution to the reactor before being cooled down to ambient temperature.

### Example 1D - Filtration of the coated nanostructures

The crude reaction mixture was transferred to a 20 I Nalgene jug. The reactor was rinsed with ultrapure water (5 + 4 liter) and the washings added to the reaction mixture. The solution was filtered through a filtration setup consisting of a 1.2 µm, a 0.45 µm, and a 0.2 µm filter connected in series, and the filters washed through with 3 liters of ultrapure water. The filtered solution was purified by ultrafiltration using a 5400 cm² 300 kD TFF filter until the concentration of ethylene glycol was below 500 ppm and then concentrated to 1.3 liters.
Average diameter (DLS) = 27.4 nm, Ð_{d} = 1.17, [P](ICP-OES) = 168 mM, [Si](ICP-OES) = 665 mM.

### Example 1E - Formulation of filtered coated nanostructures

A mixture of 423 g of the solution of coated nanostructures from Example 1D, 1.24 g of thioglycerol, 369 mg of gentisic acid, and 35.0 g of glycerol was diluted to 1154 ml with ultrapure water. The solution was filtered through a 0.2 µm polyethersulfone (PES) filter. The solution was portioned into 5 ml vials, frozen, and stored at -70 °C.

### Example 1F - Formulation of filtered coated nanostructures, with ¹⁷⁵Lu and Ca²⁺

1.41 g of thioglycerol and 425 mg of gentisic acid was added to 885 g of the solution of coated nanostructures from Example 1D. The solution was filtered through a 0.2 µm PES filter, whereafter 8.76 g of a 747 mM solution of LuCl₃ in water was added and the solution was heated in a 63.5 °C water bath for 105 minutes. To the solution was added 92.6 g of a 2 M tris buffer with a pH of 8.9. It was then heated in a 63.5 °C water bath for 120 minutes, and cooled down in an ice bath. To the solution was added 25.2 g of a 1422 mM solution of CaCl₂ and 38.7 g of glycerol at ambient temperature. It was then diluted with 243 g water and filtered through a 0.2 µm PES filter. The solution was portioned into 5 ml vials, frozen, and stored at -70 °C.

### Example 2 - Synthesis of nanostructures

Nanostructures according to the present disclosure were synthesized, filtered and formulated as described below. This example describes how to prepare nanomaterials with average size towards the low end of the current disclosure, at a small scale.

### Example 2A - Synthesis of the core nanostructure

6.06 g of 1,7-bis(triethoxysilyl)-4,4-bis(dimethylphosphonato)heptane of a purity of 96%, 248 ml of 80 % (v/v) aqueous ethylene glycol and 20.3 mg rhodamine isothiocyanate in 1.74 ml ethylene glycol were mixed in a 500 ml round bottom flask. The solution was degassed and portioned into 19 vials. The vials were capped with septa and heated to 125 °C in an oil bath for 302 hours. The solution from all vials were pooled and filtered through a glass fiber filter.
Average diameter (DLS) = 12.5nm, Ð_{d} = 1.25, [P](ICP-OES) = 78 mM, [Si](ICP-OES) = 87 mM.

### Example 2B - Addition of the anchoring layer

A 10 ml sample of the solution of precursor nanostructures from Example 2A was charged in a vial and heated to 100 °C in an oil bath. After 15 minutes 315 mg of bis(triethoxysilyl)methane was added with vigorous stirring. The heating was maintained for 4 hours with more moderate stirring.
Average diameter (DLS) = 13.5 nm, Ð_{d} = 1.3. , [P](ICP-OES) = 72 mM, [Si](ICP-OES) = 269 mM

### Example 2C - Addition of the coating layer

A 1.75 ml sample of the solution of primed nanostructures from Example 2B was diluted to 5 ml with 80 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 10 minutes in a preheated 110 °C oil bath before 60 µl of a 50 % (w/w) solution of 1,7-bis(triethoxysilyl)-4,4-bis(ω-methyl-(ethyleneoxy)₄₅-methyl)heptane in anhydrous dioxane heated to 40 °C was added. A further 510 µl of the warm 1,7-bis(triethoxysilyl)-4,4-bis(ω-methyl-(ethyleneoxy)₄₅-methyl)heptane solution was added by syringe pump at a speed of 27 µl/hour. After a total of 48 hours the solution was allowed to cool to ambient temperature.

### Example 2D - Filtration of the coated nanostructures

The resulting nanostructure solution from Example 2C was diluted to 25 ml with water and filtered through a 0.2 µm polyethersulfone (PES) syringe filter. The resulting solution was diluted to 800 ml with water and concentrated to approximately 20 ml using a tangential flow filtration setup with a 300 kD nominal cut off. The dilution - concentration procedure was repeated for a total of four times before the solution was further concentrated to 4.3 ml on a spin filter with a 300 kD nominal cut off.
Average diameter (DLS) = 22.9nm, Ð_{d} = 1.25, [P](ICP-OES) = 27 mM, [Si](ICP-OES) = 102 mM.

### Example 3 - Synthesis of nanostructures

Nanostructures according to the present disclosure were synthesized, filtered and formulated as described below. This example shows how to prepare nanostructures under pressure which gives flexibility in the choice of solvent for a desired temperature.

### Example 3A - Synthesis of the core nanostructure

A 250 ml bomb vial was charged with 11.35 g of 1,7-bis(triethoxysilyl)-4,4-bis(dimethylphosphonato)heptane of a purity of 96% and 175 ml of 80 % (v/v) aqueous ethylene glycol. The solution was degassed and put under an atmosphere of nitrogen. The bomb vial was closed off and heated to 125 °C in an oil bath for 307 hours. The solution was filtered through a glass fiber filter. Mean diameter 23.0 nm. Ð_{d} = 1.28. [P](ICP-OES)= 187 mM, [Si](ICP-OES)= 214 mM.

### Example 3B - Addition of the anchoring layer

A 10 ml sample of the solution of precursor nanostructures from Example 3A was charged in a vial and heated to 100 °C in an oil bath. After 20 minutes, 809 mg of bis(triethoxysilyl)methane was added with vigorous stirring. The heating was maintained for 4 hours with more moderate stirring.
Average diameter (DLS) = 27.3 nm, Ð_{d} = 1.24.

### Example 3C - Addition of the coating layer

A 1.025 ml sample of the solution of primed nanostructures from Example 3B was diluted to 7.5 ml with 80 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 10 minutes in a preheated 110 °C oil bath before 200 µl of a 50 % (w/w) solution of 1,7-bis(triethoxysilyl)-4,4-bis(ω-methyl-(ethyleneoxy)₄₅-methyl)heptane in anhydrous dioxane heated to 40 °C was added. A further 805 µl of the warm 1,7-bis(triethoxysilyl)-4,4-bis(ω-methyl-(ethyleneoxy)₄₅-methyl)heptane solution was added by syringe pump at a speed of 43 µl/hour. After a total of 48 hours the solution was allowed to cool to ambient temperature.

### 3D - Filtration of the coated nanostructures

The resulting nanostructure solutions from two identical batches were combined and diluted to 50 ml with water and filtered through a 0.2 µm polyethersulfone (PES) syringe filter. The resulting solution was diluted to 800 ml with water and concentrated to approximately 20 ml using a tangential flow filtration setup with a 300 kD nominal cut off. The dilution - concentration procedure was repeated for a total of five times before the solution was further concentrated to 11.3 ml on a spin filter with a 300 kD nominal cut off.
Average diameter (DLS) = 35.3 nm, Ð_{d} = 1.24, [P](ICP-OES) = 28 mM, [Si](ICP-OES) = 123 mM.

### Example 4 - Synthesis of core nanostructures with different sizes

Solutions of 1,7-bis(triethoxysilyl)-4,4-bis(dimethylphosphonato)heptane in 80 % (v/v) aqueous ethylene glycol with concentrations of 34, 37, 41, 45, 50, 55, 61, 67, 75, and 82 mM were prepared by dilution from a stock solution. 10 ml samples of each solution were charged in reaction vials and heated to 125 °C in an oil bath for 402 hours. The resulting nanostructures had diameters of 13.5, 14.3, 15.4, 15.3, 16.1, 17.4, 17.7, 20.5, 22.6, and 23.9 nm, respectively.

### Example 5 - Synthesis of primed core nanostructures with anchoring layers of different thickness

Core nanostructures synthesized according to Example 2A were treated with 2.5, 3, 5 and 7 molar equivalents of bis(triethoxysilyl)methane relative to the 1,7-bis(triethoxysilyl)-4,4-bis(dimethylphosphonato)heptane used to synthesize the core nanostructures, in a procedure similar to the one used in Example 2B. The resulting primed nanostructures had anchoring layer thicknesses of 0.5, 0.6, 0.75 and 0.95 nm.

### Example 6 - Synthesis of nanostructures using different coatings

### Example 6a - Synthesis of nanostructures coated with 1,7-bis(triethoxysilyl)-4A-bis(ω-methyl-(ethyleneoxy)₆₇-methyl)-heptane

1,7-bis(triethoxysilyl)-4,4-bis(ω-methyl-(ethyleneoxy)₆₇-methyl)heptane was synthesized analogously to 1,7-bis(triethoxysilyl)-4,4-bis(ω-methyl-(ethyleneoxy)₄₅-methyl)heptane.

A 13.4 ml sample of a solution of primed nanostructures according to Example 1B was diluted to 36.1 ml with 90 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 20 minutes in a preheated 110 °C oil bath before 1500 µl of a 50 % (w/w) solution of 1,7-bis(triethoxysilyl)-4,4-bis(ω-methyl-(ethyleneoxy)₆₇-methyl)heptane in ethanol heated to 40 °C was added. A further 5200 µl of the warm 1,7-bis(triethoxysilyl)-4,4-bis(ω-methyl-(ethyleneoxy)₆₇-methyl)heptane solution was added by syringe pump at a speed of 266 µl/hour. After a total of 48 hours the solution was allowed to cool to ambient temperature.

The resulting nanostructure solutions was diluted to 150 ml with water and filtered through double glass fiber filters and subsequently through a 0.2 µm polyethersulfone (PES) syringe filter. The resulting solution was diluted to 800 ml with water and concentrated to approximately 20 ml using a tangential flow filtration setup with a 70 kD nominal cut off. The dilution - concentration procedure was repeated for a total of 4 times before the solution was further concentrated to 20 ml on a spin filter with a 300 kD nominal cut off.

Nanostructures synthesized in this manner do not aggregate on exposure to 20 mM CaCl₂.
Average diameter (DLS) = 32.5 nm, Ð_{d} = 1.38, [P](ICP-OES) = 45 mM, [Si](ICP-OES) = 233 mM.

### Example 6b - Synthesis of nanostructures coated with 1,7-bis(triethoxysilyl)-4,4-bis(ω-methyl-(ethyleneoxy)₉₁-methyl)-heptane

1,7-bis(triethoxysilyl)-4,4-bis(ω-methyl-(ethyleneoxy)₉₁-methyl)heptane was synthesized analogously to 1,7-bis(triethoxysilyl)-4,4-bis(ω-methyl-(ethyleneoxy)₄₅-methyl)heptane.

A 4.1 ml sample of a solution of primed nanostructures according to example 1B was diluted to 11 ml with 90 % (v/v) aqueous ethylene glycol in a 2-necked round bottom flask with the vertical neck equipped with a reflux condenser topped connection to a vacuum-nitrogen manifold and the side neck stoppered with a septum. The solution was heated for 15 minutes in a preheated 110 °C oil bath before 530 µl of a 50 % (w/w) solution of 1,7-bis(triethoxysilyl)-4,4-bis(ω-methyl-(ethyleneoxy)₉₁-methyl)heptane in ethanol heated to 40 °C was added. A further 2120 µl of the warm 1,7-bis(triethoxysilyl)-4,4-bis(ω-methyl-(ethyleneoxy)₉₁-methyl)heptane solution was added by syringe pump at a speed of 109 µl/hour. After a total of 48 hours, the solution was allowed to cool to ambient temperature.

The resulting nanostructure solutions was diluted to 40 ml with water and filtered through double glass fiber filters and subsequently through a 0.2 µm polyethersulfone (PES) syringe filter. The resulting solution was diluted to 800 ml with water and concentrated to approximately 20 ml using a tangential flow filtration setup with a 70 kD nominal cut off. The dilution - concentration procedure was repeated for a total of 4 times before the solution was further concentrated to 10 ml on a spin filter with a 300 kD nominal cut off.

Nanostructures synthesized in this manner do not aggregate on exposure to 20 mM CaCl₂.
Average diameter (DLS) = 41.2 nm, Ð_{d} = 1.59, [P](ICP-OES) = 24 mM, [Si](ICP-OES) = 116 mM.

### Example 7 - Determination of elemental composition and average number of bonds per monomer of the nanostructure

Small molecule components of a sample of nanostructures according to Example 1E were removed through 10 cycles of concentration through spin filtration - dilution, using a 10 kDa cut-off spin filter. [P] and [Si] was determined through ICP-OES. The bulk of the sample was freeze dried until constant weight, and analyzed for [C], [P] and [H] by combustion analysis.

**Table 1. Elemental composition of freeze-dried samples of the nanostructures from Example 1E.**

| **Element** | **Weight %** |
|---|---|
| C | 30.45 |
| H | 6.01 |
| P | 5.41 |
| Si | 17.66 |

A model where the molar fractions of the components C₇H₁₂P₂Si₂O₉, CH₂Si₂O₃, C₁₉₀H₃₇₃Si₂O₉₁, H₂O, and C₂H₄O₂, were fitted to the empirical elemental composition. The formulas C₇H₁₂P₂Si₂O₉, CH₂Si₂O₃, and C₁₉₀H₃₇₃Si₂O₉₁ represent theoretical monomer residues with 6 bonds to the polymeric network derived from 1,7-bis(triethoxysilyl)-4,4-bis(dimethyl-phosphonato)heptane, bis(triethoxysilyl)methane, and 1,7-bis(triethoxysilyl)-4,4-bis(ω-methyl-(ethyleneoxy)₄₅-methyl)-heptane, respectively. Each H₂O represent 2 dangling silanol bonds, i.e. the formal hydrolysis of a Si-O-Si group in the polymeric structure to two Si-OH groups. C₂H₄O₂ represents ethylene glycol bonded into the siloxane network.

The fitting gave the relative composition of 1 C₇H₁₂P₂Si₂O₉, 2.49 CH₂Si₂O₃, 0.101 C₁₉₀H₃₇₃Si₂O₉₁, 3.36 H₂O, and 0.14 C₂H₄O₂. This indicates an average of 4.1 bonds to the rest of the polymeric network per monomer residue, i.e. 68 % of the theoretical maximum of 6 bonds per monomer residue.

### Example 8 - NMR of nanostructures

Samples of core nanostructures similar to the ones from Example 1A and of primed nanostructures similar to the product from Example 1B were transferred to D₂O by first exchanging the solvent to water using repeated dilution-concentration cycles on a 100 kD nominal cut-off spin filter and then exchanging the water for D₂O in the same manner. A sample of coated nanostructures according to Example 1D was similarly transferred to D₂O by repeated dilution-concentration cycles on a spin filter.

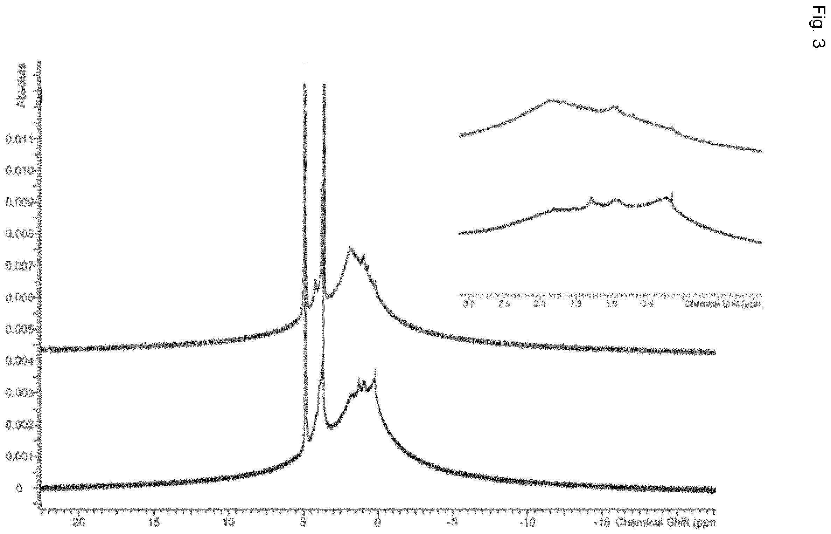

¹H-NMR spectra of core nanostructures and primed core nanostructures are shown in Fig. 3. The upper spectrum is the spectrum for the core nanostructures offset from the spectrum for the primed nanostructures for clarity. The inset displays a zoom-in of the same spectra, showing salient features in the region around 0 ppm.

The main peak for the core nanoparticles is extremely broadened, with a peak with of ca 25 ppm (12500 Hz), in accordance with a high degree of cross-linking in the core nanostructures. The spectrum for the primed nanostructures displays essentially the same features, except for a smaller but similarly broadened peak centered at 0.25 ppm corresponding to the methylene moiety in the anchoring layer. This demonstrates a high degree of cross-linking in the anchoring layer.

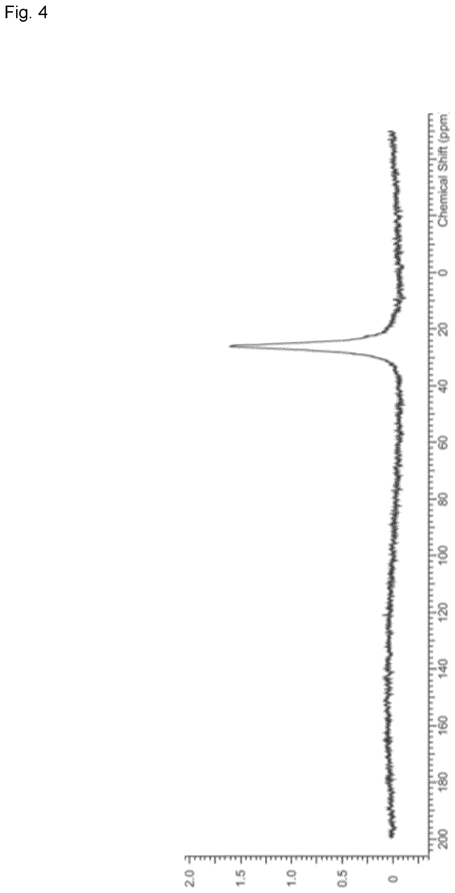

A ³¹P-NMR spectrum of the core nanostructures is shown in Fig. 4A single peak can be observed, centered around 25 ppm. That only a single chemical environment of phosphorus is present indicates the complete hydrolysis of phosphonate esters.

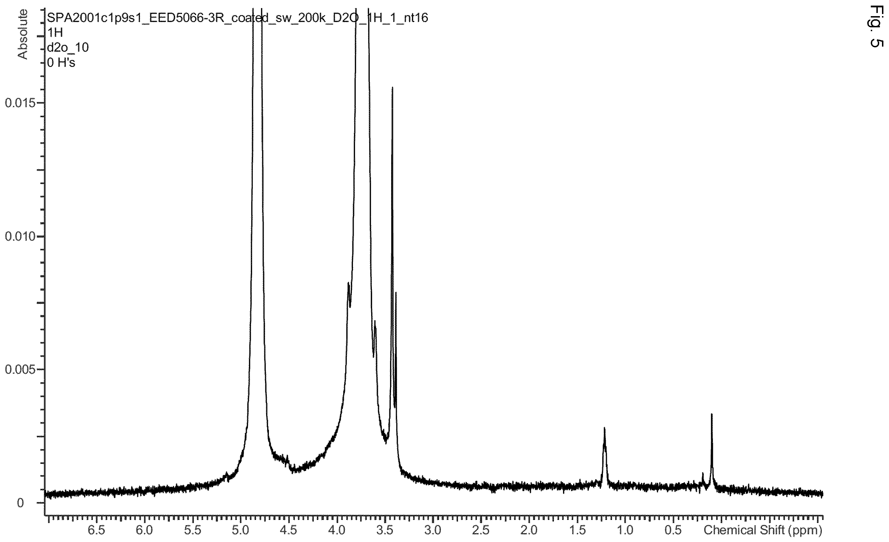

¹H-NMR spectra of the coated nanostructures is shown in Fig. 5. The spectrum is totally dominated by the PEG CH₂ signals. The signal is quite sharp which indicates that the PEG chains are rather mobile which is in line with the attachment at the surface at a single point.

### Example 9 - Comparison of loading of nanostructures by the method of the current disclosure with conventional loading

Samples of nanostructures according to Example 1E were loaded with LuCl₃ by the method of the present disclosure (low pH) and by a reference method (at neutral pH), and Lu binding strength was evaluated through competitive binding with the reference chelator EDTA.

For the low pH loading procedure, a 1000 µl sample of nanostructure solution, with a pH of 2.3, was mixed with 115 µl of a 21 mM solution of LuCl₃ and incubated at 60 °C for 1 hour. The pH was then adjusted by addition of 145 µl of a 2 M tris buffer with pH 8, before incubating for a further 2 hours at 60 °C.

For the neutral pH loading procedure, a 1000 µl sample of nanostructure solution was adjusted to a pH of 7.4 by the addition of 130 µl of a 2 M tris buffer with pH 8. The buffered nanostructure solution was then mixed with 115 µl of a 21 mM solution of LuCl₃ and incubated at 60 °C for 1 hour.

The LuCl₃ loaded nanostructure solutions were diluted to 0.2 mM Lu with 50 mM Tris buffer and treated with EDTA (10 equivalents to Lu) before incubating for 1 h at 37 °C. After incubation, an aliquot of each sample was set aside for [Lu] determination by ICP-OES without filtration and a separate aliquot of each sample filtered through a 3 kDa spin filter for determination of [Lu] not bound to the nanostructures by ICP-OES of the permeate. The relative amount of Lu not bound to the nanoparticle was calculated as [Lu]ₚₑᵣₘₑₐₜₑ/[Lu]_{unfiltered}. For the nanostructures loaded by the method of the present disclosure, 6.6% of the loaded lutetium could be extracted by EDTA, while for the reference method more than 2.5 times that amount, 17.1%, of the loaded lutetium could be extracted. Percentages are averages of two independent experiments.

This demonstrates the stronger metal capacity of nanostructures loaded with metal ions through the method of the present disclosure.

### Example 10 - Examples of loading nanostructures with cold equivalent of radionuclides

Samples of nanostructures according to Example 1E were loaded with SmCl₃, YCl₃, GdCl₃, EuCl₃, TbCl₃, ZrCl₄, HoCl₃, PrCl₃, and Ga(NO₃)₃, respectively, through treating 1 ml samples of the nanostructure solution with 2.4 µmole of aqueous solutions of the respective salts, incubating the resulting mixture at 60 °C for 1 hour, adjusting the pH to ~7.2 with 2 M Tris buffer and incubating the resulting mixtures for at 60 °C 2 hours. [P] and [M] was determined for each sample by ICP-OES and an aliquot of each sample was filtered through a 3kDa spin filter and the amount of metal not bound to the nanostructure was measured as [M] in the permeate.

**Table 2. Total and free concentrations of a selection of cold equivalents to representative radionuclides. L.O.D = limit of detection, L.O.Q = limit of quantification. For samples where [M] could not be determined in the permeate the upper bound on the percentage of free M is calculated using the concentration of the most dilute ICP standard that gave an acceptable signal to noise ratio.**

| **Element** | **[M] unfiltered (mM)** | **[M] permeate (mM)** | **% free M** |
|---|---|---|---|
| Sm³⁺ | 1.9 | < L.O.D | <0.03% |
| Y³⁺ | 2.0 | < L.O.Q | <0.07% |
| Gd³⁺ | 1.9 | 0.00024 | 0.01% |
| Eu³⁺ | 2.0 | 0.00011 | 0.006% |
| Tb³⁺ | 1.9 | 0.00055 | 0.03% |
| Zr⁴⁺ | 1.9 | 0.00021 | 0.01% |
| Ho³⁺ | 2.2 | < L.O.D | <0.004% |
| Pr³⁺ | 1.8 | < L.O.D | <2% |
| Ga³⁺ | 1.3 | 0.00050 | 0.04% |

From Table 2 it is clear that all tested cold equivalents of radionuclides bind to the nanostructures strongly.

### Example 11 - Comparison of metal binding stability with and without sodium metabisulphite

Excipients were added to samples of nanostructures according to Example 1D prior to loading with LuCl₃ and the Lu binding strength of the nanostructures was evaluated through competitive binding with the reference chelator EDTA.

Five samples of nanostructures according to Example 1D comprising no additive, 3.3% glycerol, 3.3% sodium metabisulphite, 0.3% thioglycerol, and 0.03% gentisic acid, respectively, were loaded with Lu in the form of LuCl₃ to a ratio of Lu to P of 25:1. The samples were diluted to 0.2 mM Lu with 50 mM Tris buffer and treated with EDTA (10 equivalents to Lu) before incubating for 48 hours at 37 °C. After incubation an aliquot of each sample was set aside for [Lu] determination by ICP-OES without filtration and a separate aliquot of each sample filtered through a 3 kDa spin filter for determination of [Lu] not bound to the nanostructures by ICP-OES of the permeate. The relative amount of Lu not bound to the nanoparticle was calculated as [Lu]ₚₑᵣₘₑₐₜₑ/[Lu]_{unfiltered}. For the samples treated with glycerol, thioglycerol, or gentisic acid, the fraction of the loaded lutetium that could be extracted by EDTA was essentially identical to the amount that could be extracted from the sample without additives - 16% for all samples with additives, 17% for the sample without. However, for the sample treated with sodium metabisulphite, 39% of the loaded Lu could be extracted from the nanostructures by EDTA. This clearly illustrates the advantage of using thioglycerol and/or gentisic acid over sodium metabisulphite as an antioxidant for nanostructures of the current disclosure.

### Example 12 - Biodistribution study of ¹⁷⁵Lu loaded nanostructures in a tumor-bearing mouse model

### Representative preparation of test item: Test item C

A 4 ml sample of nanostructure solution from Example 4D (112 µmole P) was treated with 228 µl of a 19.7 mM LuCl₃ solution (4.5 µmole Lu) and heated to 60 °C for 1 hour before 1.269 ml of 1 M Tris buffer (pH=7.47) was added. After a further 2 hours at 60 °C, the solution was filtered through a 0.2 µm PES syringe filter.

A 3.47 ml sample of the resulting nanostructure solution was mixed with 3.80 ml saline solution and 140 µl of a 99 mM CaCl₂ solution before pH was adjusted to 7.2 with 12 µl of a 1 M NaOH solution. The solution was diluted to a total volume of 7.5 ml with saline before the solution was filtered through a 0.2 µm PES syringe filter.
Mean size (DLS) = 36.0 nm. [P](ICP-OES) = 9.9 mM, [Si](ICP-OES) = 46 mM, [Lu](ICP-OES) = 0.39 mM

### Test items A and B

Test item A and B were prepared similarly starting from nanostructure solutions according to Examples 2D and 1D, respectively.
Test item A: Mean size (DLS) = 21.3 nm. [P](ICP-OES) = 8.9 mM, [Si](ICP-OES) = 48 mM, [Lu](ICP-OES) = 0.39 mM
Test item B: Mean size (DLS) = 27.7 nm. [P](ICP-OES) = 8.7 mM, [Si](ICP-OES) = 41 mM, [Lu](ICP-OES) = 0.39 mM

### Biodistribution

The tissue distribution of the test items after intravenous injection in a tumor-bearing mouse was investigated. Injection test items were analyzed by ICP-OES for lutetium and silicon content. The obtained values for lutetium represent the total amount (i.e. 100 %) of lutetium injected.

Tumors were induced in female BALB/c mice by inoculation with 4T1 cells (1 × 10⁵ cells) injected subcutaneously in the right flank. 18 days after inoculation the tumor volume was 200-300 mm³, and the mice were distributed into groups.

Each test item was administered intravenously at 2 µmol Lu/kg and 5 ml/kg. Animals were divided into 4 groups per test item (n=5 mice/group) and groups were sacrificed at 6 hours, 24 hours, 48 hours and 168 hours post-injection. Three animals were used as control animals and were not dosed.

Blood samples were taken at the end of the study period and plasma was prepared by removal of blood cells. After termination, organs were harvested (liver, tumor and femoral muscle). Plasma samples and digested tissue samples were analyzed by ICP-OES for lutetium content, shown in Table 3.

**Table 3. % of the injected dose (% ID) Lu after 6 h, 24 h, 48 h and 168 h. "N.M." = not measured.**

| **Organ** | **Test item** | **6 h** | **24 h** | **48 h** | **168 h** |
|---|---|---|---|---|---|
| Plasma | A | 80.2 ± 6.5 | 54.7 ± 3.0 | 24.3 ± 5.0 | N.M. |
| | B | 81.0 ± 2.5 | 51.7 ± 4.9 | 23.4 ± 2.3 | N.M. |
| | C | 82.2 ± 4.5 | 41.7 ± 4.5 | 18.6 ± 2.4 | N.M. |
| Liver | A | 8.9 ± 1.0 | 16.5 ± 1.7 | 19.8 ± 2.7 | 23.0 ± 3.1 |
| | B | 7.7 ± 1.1 | 13.6 ± 1.4 | 15.7 ± 3.3 | 22.9 ± 1.8 |
| | C | 8.8 ± 0.6 | 16.1 ± 1.0 | 21.6 ± 1.8 | 25.7 ± 2.3 |
| Tumor | A | 0.8 ± 0.3 | 2.4 ± 0.6 | 2.5 ± 1.5 | 3.3 ± 1.3 |
| | B | 1.0 ± 0.4 | 2.1 ± 0.5 | 2.6 ± 1.1 | 4.2 ± 1.8 |
| | C | 1.0 ± 0.4 | 2.5 ± 1.0 | 2.5 ± 1.1 | 5.0 ± 3.8 |
| Muscle | A | 4.5 ± 2.7 | 8.8 ± 2.4 | 7.6 ± 2.3 | 8.8 ± 2.0 |
| | B | 6.2 ± 0.9 | 10.0 ± 4.2 | 11.8 ± 2.7 | 16.0 ± 7.0 |
| | C | 7.1 ± 2.4 | 10.6 ± 3.3 | 6.2 ± 1.1 | 10.4 ± 2.7 |

As can be seen in Table 3, coated nanostructures according to the present disclosure are long circulating, i.e. they have long plasma half-lives. The plasma half-lives, as calculated based on the plasma Lu concentration, were 23.9 h for test item A, 23.1 h for test item B, and 19.8 h for test item C. Additionally, it can be seen that a significant fraction of the nanostructures distributes to the tumor. This demonstrates the suitability of nanostructures according to the current disclosure for use in a pharmaceutical composition and for use in the imaging and/or therapy of cancer.

### Example 13 - Use of ¹⁷⁷Lu loaded nanostructures to treat cancer in a tumor-bearing mouse model

Tumors were induced in 10 weeks old female BALB/c mice by inoculation with 4T1 cells (1 × 10⁵ cells) injected subcutaneously in the right flank.

Nanostructures according to Example 1D were loaded with ¹⁷⁷Lu to a concentration of approximately one atom of ¹⁷⁷Lu per nanostructure by treating a solution of the nanostructures with a solution of ¹⁷⁷LuCl₃ before adjusting the pH with tris buffer, and formulated for injection by addition of CaCl₂ and saline.

When the tumor volume reached 200-300 mm³, at day 14 or 15 after inoculation, the treatment group (n=16) was injected with the ¹⁷⁷Lu loaded nanostructure at a dose of 218 MBq/kg and the control group (n=10) received saline. Anti-tumor efficacy was monitored by following tumor growth and overall survival.

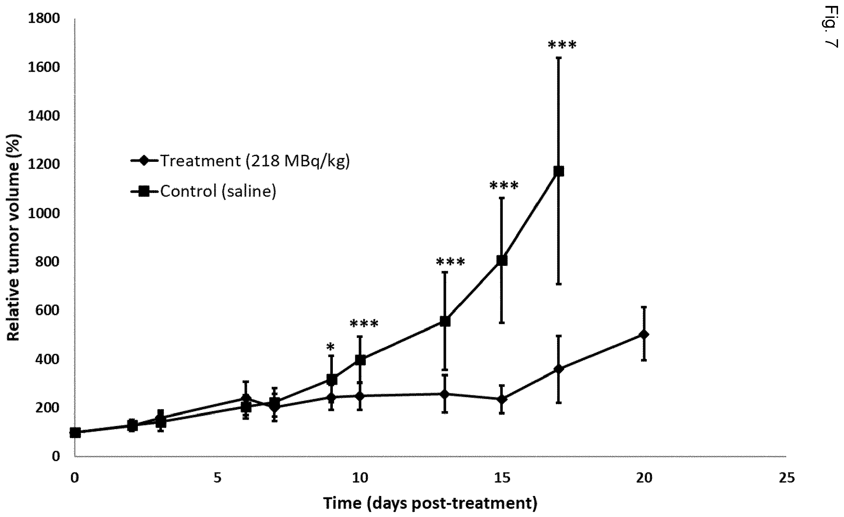

The treatment group showed significant increased mean and median survival time as compared to the control group. The tumor growth was significantly slowed down for the treatment group. See Fig. 7.

**Table 4. Median and mean survival for 4T1 tumor bearing female BALB/c mice, treated with ¹⁷⁷Lu loaded nanoparticles and control.**

| | **Median survival (days after treatment)** | **Mean survival (days after treatment)** |
|---|---|---|
| Treatment group | 13 | 14.9 |
| Control | 10 | 10.7 |

This demonstrates the use of nanostructures according to the current disclosure as intermediates in the production of a pharmaceutical composition, the use of nanostructures according to the current disclosure as intermediates in the production of a pharmaceutical composition for the use in the treatment of cancer, and the use of nanostructures according to the current disclosure as intermediates in the production of a carrier for radionuclides.

### Example 14 - Pharmacokinetics after of intravenous ¹⁷⁷Lu loaded nanostructures in healthy Sprague Dawley female and male rats

Nanostructures according to Example 1E were loaded with ¹⁷⁷Lu to a concentration of approximately one atom of ¹⁷⁷Lu per nanostructure by treating a solution of the nanostructures with a solution of ¹⁷⁷LuCl₃ before adjusting the pH with tris buffer, and formulated for injection by addition of CaCl₂ and saline.

Female and male healthy Sprague Dawley rats were injected intravenously with 8MBq/kg of the ¹⁷⁷Lu radiolabeled nanostructures. The ¹⁷⁷Lu in terminal blood was assessed from individual rats at 1, 6, 24, 72, 168, 504, and 840 h post-injection (n=5/timepoint and gender) by an automated gammacounter.

Plasma half-lives for female and male were calculated by fitting an exponential function to decay adjusted activities. t½(female)=31.1 h. t½(male)=28.5 h.

This indicates that the nanostructures are long circulating and demonstrates the use of nanostructures according to the current disclosure as intermediates in the production of a pharmaceutical composition and the use of nanostructures according to the current disclosure as intermediates in the production of a carrier for radionuclides.

### Example 15 - Concentration of a solution of nanostructures, demonstrating the high solubility in water of nanostructures

A 20ml sample of nanostructures according to example 1D was concentrated on a 300 kDa spin filter. The concentration of P was measured to 328 mM by ICP-OES, corresponding to ca 25% nanostructures in solution, w/v. The hydrodynamic diameter was measured to 27.4 nm and no aggregates were visible on ocular inspection, indicating the absence of aggregation. This demonstrates the high solubility and resistance to aggregation of nanostructures according to the present disclosure.

### Example 16 - Pharmacokinetics of ¹⁷⁵Lu loaded nanostructures in healthy mice

Nanostructures according to Example 1D were loaded with ¹⁷⁵Lu similarly to Example 1F.

Female healthy BALB/c AnN mice were injected with the nanostructures at a dose of 2 µmol Lu/kg. Blood was sampled for determination of [Si] and [Lu] at 1, 6, and 24 hours (group 1, 5 animals) and at 24, 48, and 96 hours (group 2, 5 animals). Plasma [Si] and [Lu] were determined by ICP-OES. For group 1, the fraction of Si and Lu remaining in plasma at each time point was normalized to the level at 1 hour, and for group 2 the fraction of Si and Lu remaining in plasma at each time point was normalized to the level at 24 hours. The normalized data is presented in Table 5.

**Table 5. Plasma concentrations in % of the injected dose of Lu and Si during 96 h in healthy BALB/c AnN mice.**

| **Time (h)** | **Lu** | **Si** |
|---|---|---|
| 1 | 100 | 100 |
| 6 | 72 ± 4 | 72 ± 4 |
| 24 | 40 ± 1 | 41 ± 1 |
| 48 | 20 ± 1 | 21 ± 2 |
| 96 | 5 ± 1 | 5 ± 1 |

From Table 5 it can be seen that the pharmacokinetics of the Lu and Si is identical within the level of uncertainty of the study. The co-distribution of Lu and Si strongly indicates that the Lu remains bound to the nanostructures during the duration of the experiment.

### Example 17 - Accelerated aging of nanostructures with and without excipients

Nanostructures with (formulated as in Example 1E) and without excipients (as in Example 1D, diluted to 60 mM P with water) were portioned into 1 ml vials and incubated at 60 °C in order to subject them to accelerated aging. Samples were taken out for analysis by SEC-ELSD after 7, 14 and 34 days. Samples subjected to accelerated aging showed increasing amounts of molecular impurities, assigned as polyethylene glycol comprising degradation products of the coating layer of the nanostructures. None of the samples showed signs of aggregation. The retention time (tr) of the peak corresponding to the nanostructure remained unchanged for samples formulated as in Example 1E, i.e. formulated with excipients, (Δtr/tr<0.005, below detection limit of the method used) and increased slightly for samples without excipients (formulated as in Example 1D), indicating a small decrease in particle size (Δtr/tr∼0.02), indicating a degradation of the nanoparticles.

**Table 6. Nanostructure for samples of nanostructures subjected to accelerated aging at 60 °C for samples of nanostructures formulated according to the present disclosure and nanostructures in pure water. Purity expressed as % a/a.**

| **Incubation time (days)** | **Nanostructure purity, with excipients (formulated as in Example 1E) (% a/a)** | **Nanostructure purity, without excipients (formulated as in Example 1D) (% a/a)** |
|---|---|---|
| 0 | 98.9 | 98.9 |
| 7 | 95.2 | 74.3 |
| 14 | 95 | 66.7 |
| 34 | 92.7 | 62.8 |

From Table 6 it is clear that the degradation of the nanostructures is significantly retarded for samples formulated according to the present disclosure.

### Example 18 - Storage of samples of nanostructures formulated according to the present disclosure in a frozen state

Samples of nanostructures according to Example 1D were treated with either 3% glycerol or 5% sucrose (w/v) and portioned into vials. Vials were frozen by placing them in a freezer at -20 °C, stored in the freezer for 14 days and allowed to thaw by standing at room temperature. Samples were analyzed for changes in the size profile of the nanostructures by SEC-ELSD. Results are summarized in Table 7.

**Table 7. Results from freeze-thaw experiment.**

| **Cryoprotectant** | **Changes in size profile after storage at -20 °C for 14 days** |
|---|---|
| Glycerol | None |
| Sucrose | Significant peak broadening, indicative of aggregation |

Samples treated with glycerol did not exhibit any change in size profile after freezing and storage at -70 °C, or after freezing at -70 °C and storage at - 20 °C, whereas samples comprising sucrose exhibited signs of aggregation of the nanoparticels.

### Example 19 - Synthesis of 1,7-bis(triethoxysilyl)-4,4-bis(ω-methyl-(ethyleneoxy)₄₅-methyl)-heptane

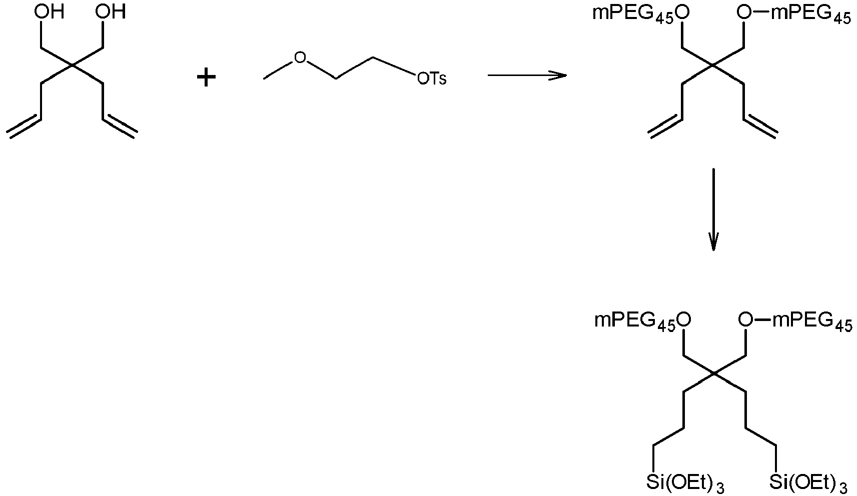

### Example 19a - Synthesis of 4,4-bis(ω-methyl-(ethyleneoxy)₄₅-methyl)-hepta-1,6-diene

Diallyl propanediol (22 g, 0.1411 mol) was dissolved in anhydrous toluene (2.61 l) and cooled in an ice bath. When the inner temperature reached below 10 °C, NaH (23.7 g, 0.593 mol, 60 % in mineral oil, 4.2 eq) was added in three portions maintaining the temperature below 10 °C. The slurry was then stirred at room temperature for 60 minutes and then added to an azeotropically dried solution of m-PEG₄₅-OTs (1.071 kg, 0.9877 mmol, 3.5 eq) in anhydrous toluene (2.61 l) under N₂ at 0 °C. The reaction mixture was heated to reflux overnight and stirred under N₂. The reaction was monitored by HPLC and upon completion, the temperature was lowered to 15 °C and the reaction was quenched by a dropwise addition of H₂O (70 ml). The pH of the crude reaction mixture was adjusted to between 5 and 7 with 1.0 M HCI (100 ml). The crude reaction mixture was split in two equal portions for practical reasons and the two portions extracted separately. Each half of the crude mixture was diluted with H₂O (7.14 l). The temperature was increased to 60 °C and NaCl (540 g) was added. The mixture was then stirred for 45 minutes and extracted three times with EtOAc (2.1 l). To the remaining aqueous phase NaCl (200 g) was added and the mixture was again extracted three times with EtOAc (2.1 l). The last three extracted fractions had an acceptable product purity (analysed by HPLC) and were dried over MgSO₄, filtered through double GF/A filters and the solvent evaporated. The resulting residues were pooled and dissolved in H₂O (2.0 l) and the pH was adjusted to pH 8 with a 0.8 M aqueous solution of NaHCO₃ (100 ml). The aqueous phase was extracted three times with DCM (500 mL). The organic phases were dried over MgSO₄, filtered and evaporated to obtain a white residue.

Consequently, the extraction process was repeated for the other half of the crude mixture and the final products of both extractions were pooled with the product of another similarly sized batch synthesized in the same manner to obtain 4,4-bis(ω-methyl-(ethyleneoxy)₄₅-methyl)-hepta-1,6-diene (758 g, 66.36 % yield, 96.8 % purity (HPLC-ELSD)).

¹H NMR (400 MHz, CDCl₃) δ 5.80 (m, 2H), 5.03 (m, 4H), 3.70-3.60 (s, 540H), 3.37 (s, 6H), 3.22 (s, 4H), 2.04 (d, 4H).

### Example 19b - Synthesis of 1,7-bis(triethoxysilyl)-4,4-bis(ω-methyl-(ethyleneoxy)₄₅-methyl)-heptane

To an azeotropically dried solution of 4,4-bis(ω-methyl-(ethyleneoxy)₄₅-methyl)-hepta-1,6-diene from Example 19a (714 g, 0.172 mol) in toluene (5.5 l), triethoxysilane (1117 g, 6.88 mol, 40 eq) was added at 22 °C under nitrogen. Karstedt's catalyst (25.34 ml, 2 % in xylenes, 1.14 mmol, 0.0066 eq) was added in 1 mL portions using a syringe over 30 minutes which resulted in an exotherm of ≤ 2 °C. The reaction mixture was left stirring at 22 °C under nitrogen overnight.

The reaction was monitored by ¹H-NMR for the disappearance of the olefinic protons. The solvent was then evaporated and excess silane was removed by co-evaporating with anhydrous toluene (2.5 l) for a total of four times. The residue was then redissolved in toluene (4.2 l), degassed with 3 cycles of vacuum/nitrogen and stirred with activated SIR-200 resin (175 g) for 3 days at 60 °C. The solution was filtered from the resin, the resin washed with toluene (3 × 2.8 l), the collected fractions were filtered through double GF/A filters, pooled and the solvent evaporated to obtain 1,7-bis(triethoxysilyl)-4,4-bis(ω-methyl-(ethyleneoxy)₄₅-methyl)-heptane as a white solid in quantitative yield (783.2 g, ≥99 %, 94.4 % purity (HPLC-ELSD)). ¹H NMR (400 MHz, C₆D₆) δ 3.71 (q, 12H), 3.70-3.40 (s, 400H), 3.52 (s, 4H), 3.35 (s, 6H), 1.68 (m, 4H), 1.59 (m, 4H), 1.23 (t, 18H), 0.79 (t, 4H).

### Example 20 - Reference nanostructures coated with the coating monomer 1-(ω-methyl-(ethyleneoxy)45-methyl)-3,5-bis(3-(triethoxysilyl)propyloxy)-benzene (Reference example outside the scope of the present disclosure)

A 8 ml sample of a solution of primed nanostructures with a hydrodynamic diameter of 15.1 nm and a phosphorus concentration of 99 mM, prepared analogously to the ones in Example 1B, was charged in a 2-necked round bottom flask with the vertical neck equipped with a reflux condenser topped connection to a vacuum-nitrogen manifold and the side neck stoppered with a septum. The solution was heated for 5 minutes in a preheated 110 °C oil bath before 300 µl of a 50 % (w/w) solution of 1-(ω-methyl-(ethyleneoxy)₄₅-methyl)-3,5-bis(3-(triethoxysilyl)propyloxy)-benzene in dioxane heated to 40 °C was added. A further 1970 µl of the warm 1-(ω-methyl-(ethyleneoxy)₄₅-methyl)-3,5-bis(3-(triethoxysilyl)propyloxy)-benzene solution was added by syringe pump at a speed of 99 µl/hour. After a total of 48 hours the solution was allowed to cool to ambient temperature.

The resulting nanostructure solution was filtered through a 0.2 µm PES filter, diluted with water and purified by ultrafiltration using a 115 cm² 300 kD TFF filter.

Nanostructures synthesized in this manner do not aggregate on exposure to 20 mM CaCl₂.

Average diameter (DLS) = 29.4 nm, Ðd = 1.33, [P](ICP-OES) = 59 mM, [Si](ICP-OES) = 387 mM.

### Example 21 - Comparison of in vivo circulation times of nanostructures according to the present disclosure and reference nanostructures

Nanostructures similar to Example 1D (test item A) and the reference nanostructures from Example 20 (test item B) were loaded with yttrium in the form of YCl₃ so that [P]/[Y] was 23 (±1). The test items were formulated for injection by adjusting [Ca], pH, and osmolality to physiological levels with CaCl₂, NaOH, and mannitol, respectively. *In vivo* behavior was investigated by injecting female BALB/c mice with the test items at a dose of 2 µmol Y/kg. Blood was sampled at 1, 6, and 24 hours after injection and plasma was analyzed for [Y] and [Si] by ICP-OES. Results in the form of circulation half-lives are shown in Table 8.

**Table 8. Plasma half-lives of nanostructures according to the present disclosureand reference nanostructures, calculated from [Y] and from [Si] as measured by ICP-OES.**

| **Test item** | **t_{½} (Y) (h)** | **t_{½} (Si) (h)** |
|---|---|---|
| A | 9.4 | 4.5 |
| B (reference) | 3.5 | 2.1 |

As can be seen in Table 8, nanostructures according to the present disclosureshow significantly prolonged circulation times, as compared to reference nanostructure not within the scope of the present disclosure.

### Example 22 - Synthesis of 1,7-bis(triethoxysilyl)-4,4-bis(ω-methyl-(ethyleneoxy)₄₅-methyl)-heptane (Compound 10)

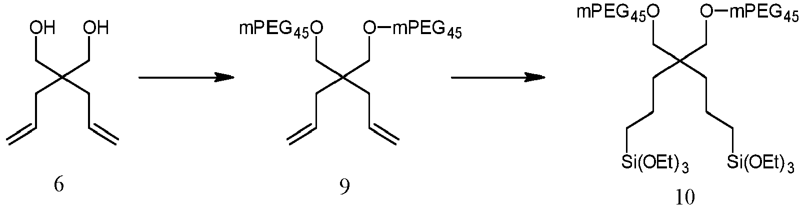

### Example 22a - Compound 9, 4,4-bis(ω-methyl-(ethyleneoxy)₄₅-methyl)-hepta-1,6-diene

Diallyl propanediol **6** (22 g, 0.1411 mol) was dissolved in anhydrous toluene (2.61 l) and cooled in an ice bath. When the inner temperature reached below 10 °C, NaH (23.7 g, 0.593 mol, 60 % in mineral oil, 4.2 eq) was added in three portions maintaining the temperature below 10 °C. The slurry was then stirred at room temperature for 60 minutes and then added to an azeotropically dried solution of m-PEG₄₅-OTs (1.071 kg, 0.9877 mmol, 3.5 eq) in anhydrous toluene (2.61 l) under N₂ at 0 °C. The reaction mixture was heated to reflux overnight and stirred under N₂. The reaction was monitored by HPLC and upon completion, the temperature was lowered to 15 °C and the reaction was quenched by a dropwise addition of H₂O (70 ml). The pH of the crude reaction mixture was adjusted to between 5 and 7 with 1.0 M HCl (100 ml). The crude reaction mixture was split in two equal portions for practical reasons and the two portions extracted separately. Each half of the crude mixture was diluted with H₂O (7.14 l). The temperature was increased to 60 °C and NaCl (540 g) was added. The mixture was then stirred for 45 minutes and extracted three times with EtOAc (2.1 l). To the remaining aqueous phase NaCl (200 g) was added and the mixture was again extracted three times with EtOAc (2.1 l). The last three extracted fractions had an acceptable product purity (HPLC) and were dried over MgSO₄, filtered through double GF/A filters and the solvent evaporated. The resulting residues were pooled and dissolved in H₂O (2.0 l) and the pH was adjusted to pH 8 with an 0.8 M aqueous solution of NaHCO₃ (100 ml). The aqueous phase was extracted three times with DCM (500 mL). The organic phases were dried over MgSO₄, filtered and evaporated to obtain a white residue.

Consequently, the extraction process repeated for the other half of the crude mixture and the final products of both extractions were pooled to obtain the desired compound **9** (758 g, 66.36 % yield, 96.8 % purity (HPLC-ELSD)). ¹H NMR (400 MHz, CDCl₃) δ 5.80 (m, 2H), 5.03 (m, 4H), 3.81 (m, 4H), 3.70-3.60 (s, 540H), 3.37 (s, 6H), 3.22 (s, 4H), 2.04 (d, 4H).

### Example 22b - Compound 10, 1,7-bis(triethoxysilyl)-4,4-bis(ω-methyl-(ethyleneoxy)₄₅-methyl)-heptane

To the azeotropically dried solution of compound **9** from Example 22a (714 g, 0.172 mol) in toluene (5.5 l), triethoxysilane (1117 g, 6.88 mol, 40 eq) was added at 22 °C under nitrogen. Karstedt's catalyst (25.34 ml, 2 % in xylenes, 1.14 mmol, 0.0066 eq) was added in 1 mL portions using a syringe over 30 minutes which resulted in an exotherm of ≤ 2 °C. The reaction mixture was left stirring at 22 °C under nitrogen overnight.

The reaction was monitored by ¹H-NMR for the disappearance of the olefinic protons. The solvent was then evaporated and excess silane was removed by co-evaporating with anhydrous toluene (2.5 l) for a total of four times. The residue was then redissolved in toluene (4.2 l) degassed with 3 cycles of vacuum/nitrogen and stirred with activated SIR-200 resin (175 g) for 3 days at 60 °C. The solution was filtered from the resin, the resin washed with toluene (3 × 2.8 l), the collected fractions were filtered through double GF/A filters, pooled and the solvent evaporated to obtain compound **10** as a white solid in quantitative yield (783.2 g, ≥99 %, 94.4 % purity (HPLC-ELSD)).

¹H NMR (400 MHz, C₆D₆) δ 3.88 (q, 12H), 3.70-3.40 (s, 400H), 3.14 (s, 6H), 1.68 (m, 4H), 1.59 (m, 4H), 1.23 (t, 18H), 0.79 (t, 4H).

## Claims

1. A plurality of globular nanostructures having an average hydrodynamic diameter between 20 to 50 nm, wherein each nanostructure is a polymeric nanostructure comprising:
a central part comprising monomer residues according to Formula (I): 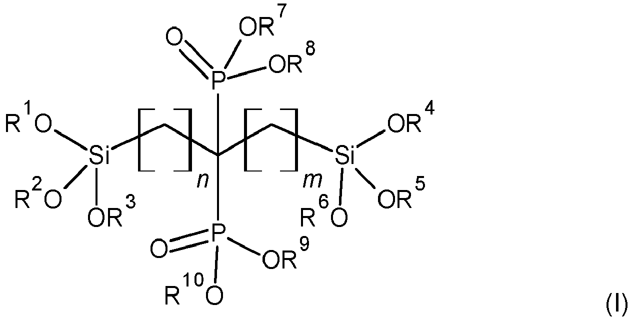 wherein
- each R¹, R², R³, R⁴, R⁵, and R⁶ is independently chosen from the group consisting of H, a negative charge, and a covalent bond;
- each R⁷, R⁸, R⁹, and R¹⁰ is independently chosen from the group consisting of H, a negative charge, and lower alkyls, wherein at least 95 % of all of the R⁷, R⁸, R⁹, and R¹⁰ groups are H or a negative charge;
- n is an integer between 2 and 5; and
- m is an integer between 2 and 5;
an anchoring layer surrounding the central part, wherein the anchoring layer comprises monomer residues according to Formula (II): 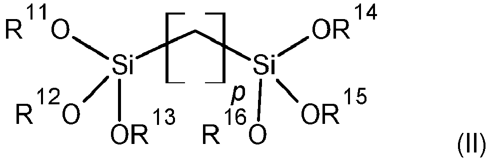 wherein
- each R¹¹, R¹², R¹³, R¹⁴, R¹⁵, and R¹⁶ is independently chosen from the group consisting of H, a negative charge, and a covalent bond; and
- p is an integer between 1 and 3;
and
a coating layer surrounding the anchoring layer, wherein the coating layer comprises monomer residues according to Formula (III): 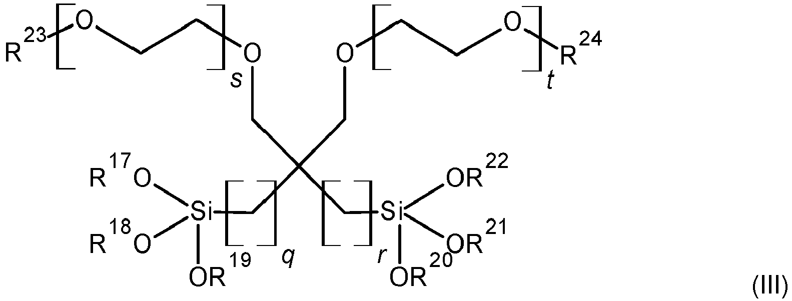 wherein
- each R¹⁷, R¹⁸, R¹⁹, R²⁰, R²¹, and R²² is independently chosen from the group consisting of H, a negative charge, and a covalent bond;
- q is an integer between 2 and 5;
- r is an integer between 2 and 5;
- each R²³ and R²⁴ is independently chosen from the group consisting of H and lower alkyls;
- s is an integer between 30 and 105; and
- t is an integer between 30 and 105.

2. A plurality of globular nanostructures according to claim 1, wherein
X_{CP}, denoting the percentage of the number of monomer residues of the central part in relation to the total number of monomer residues of the nanostructure, is between 5% and 58%;
X_{AL}, denoting the percentage of the number of monomer residues of the anchoring layer in relation to the total number of monomer residues of the nanostructure, is between 39% and 93%;
X_{CL}, denoting the percentage of the number of monomer residues of the coating layer in relation to the total number of monomer residues of the nanostructure, is between 0.75% and 4.5%; and
wherein 70% < (X_{CP} + X_{AL} + X_{CL}) ≤ 100%.

3. A plurality of globular nanostructures according to claim 1 or 2, wherein
the average hydrodynamic diameter is from 22 to 37 nm;
n = 3;
m = 3;
p = 1;
q = 3;
r = 3;
R²³ and R²⁴ are independently chosen from the group consisting of lower alkyls;
and/or
X_{CP}, denoting the percentage of the number of monomer residues of the central part in relation to the total number of monomer residues of the nanostructure, is between 20% and 40%;
X_{AL}, denoting the percentage of the number of monomer residues of the anchoring layer in relation to the total number of monomer residues of the nanostructure, is between 60% and 85%;
X_{CL}, denoting the percentage of the number of monomer residues of the coating layer in relation to the total number of monomer residues of the nanostructure, is between 1.5% and 4%; and
wherein 81.5% < (X_{CP} + X_{AL} + X_{CL}) ≤ 100%.

4. A plurality of globular nanostructures according to any one of the preceding claims, wherein at least 50 % of all of the R¹, R², R³, R⁴, R⁵, R⁶, R¹¹, R¹², R¹³, R¹⁴, R¹⁵, R¹⁶ R¹⁷, R¹⁸, R¹⁹, R²⁰, R²¹, and R²² groups are covalent bonds.

5. A plurality of globular nanostructures according to any one of the preceding claims, wherein the globular nanostructures further comprise one or more radionuclides.

6. A plurality of globular nanostructures according to claim 5 wherein the radionuclide is ¹⁷⁷Lu.

7. A plurality of globular nanostructures according to any one of the preceding claims for use as a medicament.

8. A plurality of globular nanostructures according to claim 5 or 6 for use in the treatment of cancer and/or imaging.

9. Use of a plurality of globular nanostructures according to any one of claims 1 to 4 as carriers of radionuclides.

10. A pharmaceutical composition comprising a plurality of globular nanostructures according to any one of claims 1 to 6, water, and at least one excipient.

11. The pharmaceutical composition according to claim 8, further comprising:
0.1 to 10 mg/ml thioglycerol;
0.03 to 3 mg/ml gentisic acid; and
3 to 300 mg/ml glycerol.

12. A pharmaceutical composition according to claim 10 or 11 for use as a medicament.

13. A pharmaceutical composition according to claim 10 or 11 for use in the treatment of cancer and/or imaging, wherein the pharmaceutical composition comprises a plurality of globular nanostructures according to claims 5 or 6.

14. A method for radiolabeling a plurality of globular nanostructures with a multivalent cationic radionuclide, wherein the method comprises the steps of:
a) providing a solution having a pH of below 3.5 and comprising a plurality of globular nanostructures, preferably wherein the nanostructures are nanostructures according to any one of claims 1 to 4;
b) contacting the solution of step a) with a pharmaceutically acceptable salt of the radionuclide; and
c) adjusting the pH of the solution to above 6.

15. The method of claim 14, wherein
in step a), the solution has a pH below 3; and/or
step b) further comprises, after contacting the solution of step a) with a pharmaceutically acceptable salt of the radionuclide, incubating the solution at a temperature between 40 °C and 80 °C for at least 15 minutes; and/or
in step c), the pH is adjusted by mixing the solution of step b) with an aqueous buffer having a pH above 6.

16. A kit, comprising:
an aqueous solution of globular nanostructures according to any one of claims 1 to 4, having a pH below 3.5;
an aqueous solution of a pharmaceutically acceptable buffer having a pH of at least 6; and
instructions for use, allowing for the realization of the method of radiolabeling according to claim 9 or 10;
optionally, a mixing vessel;
optionally, an apparatus for sterile filtration;
optionally, a pharmaceutically acceptable dilutant;
optionally, a centrifugal filter; and
optionally, equipment for the shielding of ionizing radiation.
